# EUROPÄISCHE PATENTANMELDUNG

(11) **EP 1 302 543 A1**
(43) Veröffentlichungstag der Anmeldung: **16.04.2003**
(21) Anmeldenummer: 02022230.3
(22) Anmeldetag: 02.10.2002
(51) Int. Cl.: C12N 15/63, C12N 15/85, C12Q 1/68, G01N 33/50

(54) **Splicing als Target zum Identifizieren neuer Wirkstoffe**

(30) Priorität: 15.10.2001 DE 10150783
(71) Anmelder: Bayer CropScience AG, 40789 Monheim (DE)
(72) Erfinder: Schreier, Peter, Prof. Dr., 50674 Köln (DE); Aichinger, Christian, Dr., 50939 Köln (DE)

(57) **Zusammenfassung**

Die vorliegende Erfindung betrifft neue Desoxyribonukleinsäure-(DNA)-Konstrukte, Vektoren und Wirtsorganismen, die diese DNA-Konstrukte enthalten und durch Einsatz in bestimmten Verfahren geeignet sind, den Verlust von korrektem Splicing (deutsch: Spleißen) anzuzeigen, sowie die Verwendung dieser transgenen Organismen bzw. Zellen zum Identifizieren neuer Wirkstoffe und schließlich Verfahren zum Auffinden neuer Wirkstoffe, die in der Lage sind, korrektes Splicing zu verhindern, sowie der Einsatz dieser Verfahren im Hochdurchsatz-Screening (High Throughput Screening, HTS) oder Ultra-Hochdurchsatz-Screening (Ultra High Throughput Screening, UHTS).

## Beschreibung

Die vorliegende Erfindung betrifft neue Desoxyribonukleinsäure-(DNA)-Konstrukte, Vektoren und Wirtsorganismen, die diese DNA-Konstrukte enthalten und durch Einsatz in bestimmten Verfahren geeignet sind, den Verlust von korrektem Splicing (deutsch: Spleißen) anzuzeigen, sowie die Verwendung dieser transgenen Organismen bzw. Zellen zum Identifizieren neuer Wirkstoffe und schließlich Verfahren zum Auffinden neuer Wirkstoffe, die in der Lage sind, korrektes Splicing zu verhindern, sowie der Einsatz dieser Verfahren im Hochdurchsatz-Screening (High Throughput Screening, HTS) oder Ultra-Hochdurchsatz-Screening (Ultra High Throughput Screening, UHTS).

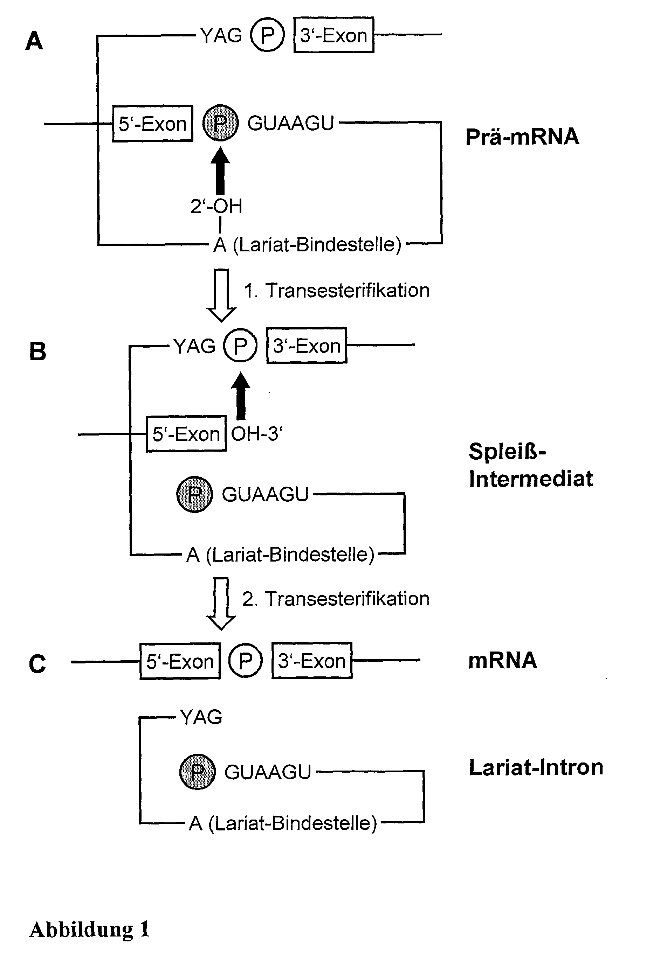

Vor mehr als 20 Jahren wurden bei Eukaryonten und ihren Viren unterbrochene Gene und das Splicing der pre-mRNA (Vorläufer-Messenger Ribonukleinsäure) entdeckt (vgl. Berget et al. (1977), Sambrook (1977)). Beim Splicing werden so genannte Introns (d.h. nicht codierende DNA-Bereiche innerhalb einer für ein Protein codierenden Sequenz) aus dem primären Gentranskript mit der Präzision eines einzigen Nucleotids herausgeschnitten. Die so genannten Exons (d.h. die codierenden DNA-Bereiche) werden dabei zusammengefügt und ergeben korrekt translatierbare mRNAs. Die Sequenz des primären Transkripts enthält konservierte Sequenzen zu Beginn, innerhalb und am Ende eines Introns, die für das Splicing mit entscheidend sind. An den äußersten Enden findet man eine konservierte Sequenz mit der Folge 5'-GU...AG-3'. Im Abstand von im allgemeinen 10 bis 40 Nucleotiden "upstream" von der 3'-Splicing-Stelle befindet sich ein Adenosin-Baustein als Verzweigungsstelle. Chemisch laufen beim Splicing nacheinander zwei Transesterifikationsreaktionen ab (siehe Abb. 1). Der erste Schritt ist die Spaltung der Grenze zwischen dem 5'-Exon und dem 3'-Rest der pre-mRNA. Dabei erfolgt ein nucleophiler Angriff der 2'-OH-Gruppe des invarianten Adenosin-Bausteins auf die 5'-Phosphatgruppe des Guanosin-Bausteins des Introns, wodurch die so genannte Lariat-Struktur entsteht (siehe Abb. 1A). Im zweiten Schritt greift dann die freie 3'-OH-Gruppe des bei der ersten Reaktion abgespaltenen Exons nun die Phosphodiesterbindung an der 3'-Splicing-Stelle an (siehe Abb. 1B). In wieweit sich das Splicing zwischen verschiedenen Organismen unterscheidet, ist nach heutigem Kenntnisstand nicht abschließend untersucht.

1985 wurde zuerst beschrieben (Grabowski et al. (1985)), dass der Splicing-Apparat (Spliceosom, deutsch: Spleißosom) mindestens so kompliziert aufgebaut ist wie ein Ribosom. Das Spliceosom besteht aus einer Anzahl von snRNAs (small nuclear RNAs) und einer Reihe von Proteinen. Die Zahl variiert jeweils von Organismus zu Organismus. In Hefe sind beispielsweise 5 snRNAs und ca. 50 Proteine beteiligt. Darüber hinaus sind etwa 100 weitere Proteine am Splicing-Prozess beteiligt. Das Spliceosom ist für mehr als 10 RNA:RNA Interaktionen in der richtigen Reihenfolge und deren zeitgerechter Wiederauflösung verantwortlich, so dass Spliceosomen ebenso wie Ribosomen hoch komplexe Ribonucleoprotein (RNP)-Maschinen sind. Das Spliceosom muss für jedes Herausschneiden eines Introns erneut zusammengesetzt werden (so genanntes Assembly). Das Zusammensetzen des Spliceosoms ist eine straff geordnete dynamische Folge von Einzelschnitten, die die Hydrolyse von mehreren ATP-Molekülen und die strukturelle Reorganisation vieler Proteine und RNA-Moleküle beinhaltet. Dieser Vorgang wird in seiner Präzision und zeitlichen Abfolge wiederum von verschiedenen anderen Proteinen gesteuert.

Eukaryonten enthalten eine große Anzahl von Genen, die gespleißt werden müssen, um zu den entsprechenden korrekten mRNAs und so zu funktionsfähigen Proteinen zu gelangen. Störungen beim Assembly des Spliceosoms sowie beim Splicing führen in aller Regel zum Zelltod.

Auf vielen Gebieten im Pflanzenschutz sowie in medizinischen Anwendungen werden immer neue Wirkstoffe gesucht, die den gestiegenen Anforderungen an Wirksamkeit, Umweltverträglichkeit, Resistenzverhalten und Kosten genügen. Unerwünschtes Wachstum von Pilzen und Unkräutern oder der Befall mit Schädlingen führen z.B. in der Landwirtschaft jedes Jahr zu beträchtlichen Schäden. Die Kontrolle dieser unerwünscht wachsenden Eukaryonten kann z.B. durch Fungizide, Herbizide oder Insektizide erfolgen. Aus den genannten Gründen besteht ein stetiger Bedarf an neuen Substanzen bzw. Substanzklassen, die zu leistungsfähigen und umweltverträglichen neuen Wirkstoffpräparaten entwickelt werden können. Im Fall von Fungiziden ist es allgemein üblich, in Gewächshaustests nach solchen neuen Leitstrukturen zu suchen. Solche Tests sind jedoch arbeitsintensiv und teuer. Die Anzahl der Substanzen, die im Gewächshaus getestet werden können, ist entsprechend begrenzt. Eine Alternative zu solchen Tests ist die Verwendung von sogenannten Hochdurchsatz-Verfahren (HTS = high throughput screening) bzw. Ultra-Hochdurchsatz-Verfahren (UHTS = ultra high throughput screening). Dabei werden in einem automatisierten Verfahren eine große Anzahl von Einzelsubstanzen hinsichtlich ihrer Wirkung auf Einzelzellen, individuelle Genprodukte oder Gene getestet. Wird für bestimmte Substanzen eine Wirkung nachgewiesen, so können diese in herkömmlichen Screening-Verfahren untersucht und gegebenenfalls weiter entwickelt werden.

Vorteilhafte Angriffspunkte für Wirkstoffe, insbesondere für Fungizide, Herbizide, Insektizide oder Pharmazeutika werden oft in essentiellen Biosynthesewegen gesucht. Beispielsweise ist ein ideales Fungizid ein solcher Stoff, der ein Genprodukt hemmt, das eine entscheidende Bedeutung bei der Ausprägung der Pathogenität von verschiedenen Pilzen hat.

Auf medizinisch-pharmazeutischem Gebiet ist bekannt, dass fehlerhaftes Splicing zu verschiedenen Krankheitsbildern führen kann. Fehlerhaftes Splicing kann bewirken, dass entscheidende Enzyme in einer inaktiven Form entstehen (Zanelli et al. (1990)). Andere Untersuchungen weisen darauf hin, dass ein defektes Genprodukt zu einer erheblichen Störung der Bildung der snRNPs führt, wodurch der Splicing-Apparat inhibiert wird (Fischer et al. (1997)). Bei der Metastasierung von Krebszellen scheinen unter anderem bestimmte alternative Splicing-Varianten eine entscheidende Rolle zu spielen (Sherman et al. (1996)).

Aus WO 00/52201 ist ein *in vitro*-Testsystem zum Erkennen einer Splicing-Reaktion bekannt. Nachteilig an diesem Testsystem ist die verhältnismäßig aufwändige Vorbereitung von Kernextrakten der verwendeten Zellen, sowie das Herstellen und Immobilisieren geeigneter Splicing-Konstrukte sowie der Nachweis der entstandenen Splicing-Produkte.

Aus WO 00/67580 ist ein weiteres *in vitro*-Verfahren zum Identifizieren von Verbindungen bekannt, die das eukaryontische Splicing beeinflussen. Nachteilig ist auch hier das notwendige Herstellen von Zellextrakten für den Einsatz in einem Splicing-Assay. Nachteilig ist außerdem, dass der Nachweis des erfolgten bzw. nicht erfolgten Splicing über gelelektrophoretische Methoden erfolgt.

Der vorliegenden Erfindung liegt nun der Ansatz zugrunde, das Spliceosom als Angriffspunkt auf der Suche nach neuen Wirkstoffen dahingehend zu verwenden, dass man mit einem geeigneten *in vivo*-Verfahren nachweisen kann, ob seine Funktion gestört, d.h. in der Aktivität verringert, oder ganz unterdrückt wird.

Unter *Spliceosom* wird hier der gesamte für das Splicing notwendige Komplex verstanden. Er setzt sich im Wesentlichen zusammen aus verschiedenen snRNAs, die in der Regel in Ribonucleoproteinpartikeln eingebunden vorliegen (so genannte snRNPs), und einer Reihe weiterer Splicing-Faktoren, u.a. RNA-bindende Proteine, die den Splicing-Vorgang unterstützen.

Das Spliceosom bietet also eine große Zahl von Angriffspunkten für Wirkstoffe unterschiedlichster Art. Neben den genannten Komponenten, aus denen das Spliceosom besteht, kommen alle Regulatoren, die für den korrekten Aufbau des Spliceosoms oder eines seiner Bausteine direkt und/oder indirekt beteiligt sind, sowie alle Faktoren, die eine zeit- und/oder ortsgerechte Expression der beteiligten Proteine bzw. das Assembly des Spliceosoms beeinflussen gut als Angriffspunkte in Frage.

Gegenstand der Erfindung ist deshalb ein Verfahren zum Nachweis der Funktionsfähigkeit des Splicing als solches, insbesondere *in vivo.*

Der Nachweis der Funktionsfähigkeit kann beispielsweise so erfolgen, dass man zunächst DNA-Konstrukte herstellt, die zusätzlich zu einer DNA-Sequenz, die alle notwendigen Elemente für ein erfolgreiches Splicing enthält (z.B. ein intaktes Intron oder eine entsprechende Sequenz), ein Reporter-Gen besitzen. Bringt man diese DNA-Konstrukte anschließend mittels Transformation, d.h. entweder mit Hilfe eines Vektors oder durch Transformation mit linearer DNA, in eine zum Splicing befähigte Wirtszelle oder einen Wirtsorganismus, wird das Reporter-Gen in Abhängigkeit von dessen Anordnung innerhalb des DNA-Konstruktes entweder bei intaktem Splicing oder bei gestörtem Splicing, bevorzugt bei Störung des Splicing, translatiert und zeigt somit die Funktionsfähigkeit des Splicing an.

Modifiziert man z.B. eine Zelle mit einem solchen DNA-Konstrukt in der Weise, dass das Reporter-Gen oder ein notwendiger Bestandteil des Reporter-Gens unter natürlichen Bedingungen herausgespleißt wird, im Falle einer Inhibierung des Splicings jedoch transkribiert und folglich translatiert wird, so lassen sich die einzelnen Fälle wie folgt unterscheiden:

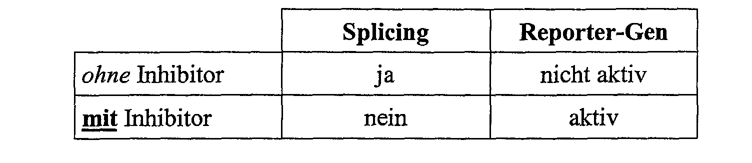

Unter *Reporter-Gen* wird hier eine codierende Sequenz mit einem leicht nachweisbaren Genprodukt oder dessen Aktivität verstanden, das mit einem geeigneten Promotor verbunden werden kann. Das Genprodukt des Reporter-Gens muss biologisch so gut charakterisiert sein, dass ein aktives Zentrum oder eine für die Funktion notwendige Proteindomäne bekannt ist (Funktionseinheit). Beispielhaft seien folgende Reporter-Gene genannt: Das für β-Galactosidase codierende *lacZ*-Gen aus *Escherichia coli;* verschiedene Luciferase-Gene, d.h. Enzyme, die Reaktionen katalysieren, die zu Biolumineszenz führen; das Gen für das GFP (green fluorescent protein) oder dessen Varianten aus der pazifischen Quallenart *Aequorea victoria*, bei dessen Translation ein fluoreszierendes Protein als Produkt entsteht.

Unter *Transformation* im Sinne der Erfindung wird ein generelles Verfahren zum Einschleusen von DNA in höhere Zellen verstanden. Dazu können entweder Vektoren verwendet werden, oder es erfolgt eine Transformation mit linearer DNA. Zur Kontrolle, ob die Transformation erfolgreich verlaufen ist, werden allgemein bekannte Selektionsmarker, wie z.B., *hph* (Hygromycin-Phosphotransferase-Gen führt zu Hygromycin B Resistenz), *npt* II (Neomycin-Phosphotransferase-Gen führt zu Kanamycin Resistenz), NAT (führt zu Resistenz gegenüber CLONAT, Hans-Knöll-Institut, Jena), *cbx* (das Gen für die Eisen-Schwefel-Untereinheit der Succinat-Dehydrogenase führt zu Resistenz gegenüber Carboxin), *pyr6* [Orotidin-5'-Monophosphat-Decarboxylase-Gen, Komplementation von *ura*^{*-*} (Δpyr6)-Stämmen] verwendet, die eine Identifikation derjenigen Zellen erlauben, in die erfolgreich fremde DNA eingeschleust wurde.

Als Vektoren können alle in molekularbiologischen Laboratorien verwendeten viralen Vektoren, Plasmide, Phasmide, Cosmide, YACs, BACs, künstliche Chromosomen oder mit DNA beschichtete Partikel, die für einen Partikelbeschuss geeignet sind, verwendet werden.

Der Ausdruck *Wirtszellen* bzw. *Wirtsorganismen,* wie er hierin verwendet wird, bezieht sich auf Zellen bzw. Organismen, die natürlicherweise die erfindungsgemäßen DNA-Konstrukte nicht enthalten. Sind beide Möglichkeiten gemeint, werden Wirtszellen und Wirtsorganismen sprachlich unter dem Begriff *Wirt* zusammengefasst.

Als Wirtszellen bzw. Wirtsorganismen eignen sich eukaryontische Zellen, wie Zellen von Pilzen, Insekten, Pflanzen, Froschoozyten und Zelllinien von Säugern, sowie ganze Organismen, wie Volvox-Kugeln, Drosophila-Embryonen oder Daphnia-Larven. Bevorzugt verwendet man transformierbare Einzelzellen, besonders bevorzugt Zellen von Pilzen, ganz besonders bevorzugt von *Saccharomyces cerevisiae, Magnaporthe grisea, Aspergillus nidulans, Cochliobulus heterostrophus, Nectria hematococca, Botrytis cinerea, Gaeumannomyces sp., Pichia pastoris* und *Ustilago maydis,* insbesondere ganz besonders bevorzugt von *Ustilago maydis*.

Gegenstand der Erfindung ist deshalb ein Verfahren zum Nachweis der Funktionsfähigkeit des Splicing, das dadurch gekennzeichnet ist, dass man Wirtszellen bzw. -organismen, die die erfindungsgemäßen DNA-Konstrukte enthalten, auf die Aktivität des Reporter-Gens untersucht. Dabei ist es unerheblich, ob bei korrektem Splicing ein messbares Signal erhalten wird oder bei Verlust bzw. eingeschränkter Aktivität des Splicings.

Gegenstand der Erfindung ist also ein Verfahren zum Nachweis der Funktionsfähigkeit des Splicing, das dadurch gekennzeichnet ist, dass man
(A) ein DNA-Konstrukt herstellt, dessen Existenz in einem eukaryontischen Organismus dazu führt, dass der Verlust von korrektem Splicing nachgewiesen werden kann;
(B) das DNA-Konstrukt aus Schritt (A) in eine Wirtszelle oder einen Wirtsorganismus einbringt;
(C) die Anwesenheit oder Abwesenheit des Reporter-Gen-Produktes kontrolliert, um die Splicing-Aktivität nachzuweisen.

Die *DNA-Konstrukte*, die in Schritt (A) hergestellt werden, müssen mehrere Eigenschaften besitzen, damit bei Verlust des korrekten Splicing ein nachweisbares Resultat vorliegt. Die einzelnen Bestandteile dieser Konstrukte sind ein Promotor, der in Eukaryonten aktiv ist, eine Intron-Sequenz, d.h. eine DNA-Sequenz, die alle funktionellen Elemente eines Introns besitzt, und ein Reporter-Gen, wobei alle diese Bestandteile unabhängig von ihrer Anordnungsreihenfolge funktionell verknüpft sind bzw. werden.

*Funktionell verknüpft* bedeutet dabei im Sinne der Erfindung, dass die Entstehung des Reporter-Gen-Produktes entweder bei intaktem oder bei gestörtem/inhibierten oder bei ganz unterdrücktem Splicing ermöglicht wird.

Die DNA-Konstrukte stellen somit Reporter bereit, die entweder bei funktionierendem Splicing-Vorgang oder bei gestörtem bzw. ganz unterdrücktem Splicing-Vorgang keine biologische Aktivität mehr besitzen. Damit kann in jedem Fall die Anwesenheit oder Abwesenheit der Aktivität nachgewiesen werden. Bevorzugt verwendet man DNA-Konstrukte, deren Reporter bei gestörtem bzw. ausgeschaltetem Splicing-Vorgang ein nachweisbares Signal liefert. Dabei ist es unerheblich, ob bereits das Assembly des Spliceosoms verhindert wird oder erst das Splicing selbst.

Als Promotoren kommen alle eukaryontischen Promotoren infrage, die die Transkription des Reporter-Gens ermöglichen. Verwendbar sind konstitutive, regulierbare bzw. synthetische Promotoren, die im jeweiligen Testsystem das DNA-Konstrukt mit dem Reporter-Gen transkribieren.

In besonders bevorzugten Ausführungsformen setzt man Promotoren von *Ustilago maydis* ein, ganz besonders bevorzugt den regulierbaren *crg1*-Promotor (Bottin et al. (1995)), den konstitutiv aktiven *hsp70*-Promotor (Holden et al. (1989)) oder den synthetischen *otef*-Promotor (Spellig et al. (1996)) oder den synthetischen *oma*-Promotor (mit der Sequenz entsprechend SEQ ID NO. 14).

*Konstitutive Promotoren* sind solche Promotoren, die eine gering regulierte, kontinuierliche Transkription von RNA ermöglichen.

*Regulierbare Promotoren* sind solche Promotoren, deren Aktivität durch spezifische Faktoren so gesteuert werden kann, dass die Transkriptionsrate gesteigert bzw. verringert werden kann.

*Synthetische Promotoren* sind solche Promotoren, die nicht natürlich vorkommen, da sie aus verschiedenen Promotoren oder Promotorfragmenten bzw. deren regulatorischen Elementen zusammengesetzt sind. Sie können die Eigenschaften konstitutiver und regulierbarer Promotoren beinhalten.

Das erfindungsgemäße DNA-Konstrukt enthält weiterhin eine *Intron-Sequenz.* Hierunter wird im Rahmen dieser Erfindung eine DNA-Sequenz verstanden, die alle Merkmale eines Introns aufweist, vom Spliceosom als Intron erkannt und bei intaktem Splicing aus der DNA herausgeschnitten wird.

Zu den notwendigen Erkennungsmerkmalen gehört eine 5'-Splicing-Stelle beginnend mit den Nucleotiden 5'-GU-3', bevorzugt beginnend mit 5'-GUAAGU-3'. Weiterhin wird eine 3'-Splicing-Stelle benötigt beginnend mit den Nucleotiden 5'-AG-3', bevorzugt mit 5'-YAG-3', wobei Y für eine Pyrimidinbase (Thymidin oder Cytosin) steht.

Daneben kann optional vor dem 3'-Ende der Intron-Sequenz eine so genannte Lariat-Bindungsstelle in Form eines Adenosin-Nucleotids (wie bei Hefen, z.B. *Saccharomyces cerevisiae*) vorliegen. In der Regel liegt im Abstand von im allgemeinen 10 bis 40 Nucleotiden (bei Hefen 14 bis 18 Nucleotide) "upstream" von der 3'-Splicing-Stelle tatsächlich ein Adenosin-Baustein als Verzweigungsstelle vor. Dieser Bereich ist nach heutigem Kenntnisstand nicht genau definiert und kann - je nach Organismus - in einem größeren Bereich variieren, der auch über die Grenzen des genannten Bereichs hinaus geht. Für die Durchführung des erfindungsgemäßen Verfahrens ist diese Bindungsstelle nicht zwingend erforderlich.

Bevorzugt werden solche Intron-Sequenzen eingesetzt, die keine Start- und/oder Stoppcodons enthalten, damit die Translation des gesamten DNA-Konstruktes durch diese Codons nicht negativ beeinflusst wird.

Erfindungsgemäß werden auch solche Intron-Sequenzen umfasst, die entsprechend modifiziert wurden, um den oben genannten Anforderungen zu genügen. Besonders trifft dies auf Modifikationen zu, bei denen eventuell vorhandene Start- und Stoppcodons entfernt werden, die die Translation vorzeitig initiieren oder beenden. Erfindungsgemäß von Interesse sind ebenfalls Variationen der 5'- bzw. 3'-Splicingsites, die eine verbesserte Effizienz des Spleißens (z.B. durch Verbesserung der Bindung des Spliceosoms an die pre-mRNA) bewirken.

Unter einem *modifizierten* Intron wird im Sinne der Erfindung ein Intron verstanden, das nach einer geeigneten Veränderung, die auch eine Mutation, z.B. eine Punktmutation sein kann, seine Funktion als Intron behält, d.h. vom Spliceosom als solches erkannt und herausgespleißt wird. Ein solches Intron wird hier auch als *funktionelles* Intron bezeichnet.

Im Gegensatz dazu, wird im Sinne der Erfindung unter einem *mutierten* Intron, ein solches Intron verstanden, das nach einer Veränderung seine Funktion als Intron verloren hat (*funktionsloses* Intron), d.h. vom Spliceosom nicht mehr erkannt und somit nicht herausgespleißt wird.

Ebenso werden erfindungsgemäß solche Intron-Sequenzen umfasst, die auf Basis verschiedener bekannter Sequenzen zusammengesetzt wurden und die Eigenschaften eines Introns behalten haben. Dies gilt besonders für Introns, deren Konsensussequenzen aus der Analyse des *Ustilago maydis*-Genoms abgeleitet wurden. Bekannte Introns, die erfindungsgemäß verwendet werden können, sind z.B. aus *U. maydis* die vier Introns des *lga2*-Gens oder die drei Introns aus dem *pra1*-Gen (Urban et al. (1996), Bölker et al. (1992)).

Bevorzugt verwendet man die Intron-Sequenz des modifizierten Introns gemäß der SEQIDNO. 10.

Als Reporter-Gene kommen in Frage: GFP und Varianten bzw. Derivate (z.B. eGFP, yGFP, cGFP), lacZ, LUX, GUS, CAT, Orotidin 5'-Monophosphatdecarboxylase, Nitrat Reduktase.

Der Aufbau der erfindungsgemäßen DNA-Konstrukte, d.h. die Reihenfolge, in welcher die einzelnen Komponenten angeordnet sind, hängt von den verwendeten Bausteinen (Promotor, Intron-Sequenz, Reporter-Gen) ab. So ist es im allgemeinen möglich, die Intron-Sequenz vor dem Reporter-Gen oder innerhalb des Reporter-Gens anzuordnen. Bevorzugt wird nach dem Promotor die Intron-Sequenz gefolgt vom Reporter-Gen angeordnet. Es ist jedoch auch möglich, das DNA-Konstrukt so herzustellen, dass die Intron-Sequenz mit dem Reporter-Gen überlappt. In einer ganz besonders bevorzugten Ausführungsform wird dabei der Übergang von der Intron-Sequenz zum Reporter-Gen so gestaltet, dass das Startcodon für das Reporter-Gen 6 Basenpaare vor der funktionellen Einheit dieses Gens beginnt und das folgende Reporter-Gen somit eine zusätzliche Aminosäure enthalten kann.

Die in den erfindungsgemäßen DNA-Konstrukten verwendeten Bausteine (Promotor, Intron-Sequenz, Reporter-Gen) sind bzw. werden unabhängig von ihrer Anordnungsreihenfolge funktionell verknüpft.

Gegenstand der Erfindung sind also weiterhin DNA-Konstrukte, deren Existenz in einem eukaryontischen Organismus dazu führt, dass der Verlust von korrektem Splicing nachgewiesen werden kann.

Gegenstand der Erfindung sind bevorzugt DNA-Konstrukte bestehend aus einem Promotor, der in Eukaryonten aktiv ist, einer DNA-Sequenz, die alle vorstehend genannten funktionellen Elemente eines Introns besitzt, und einem Reporter-Gen.

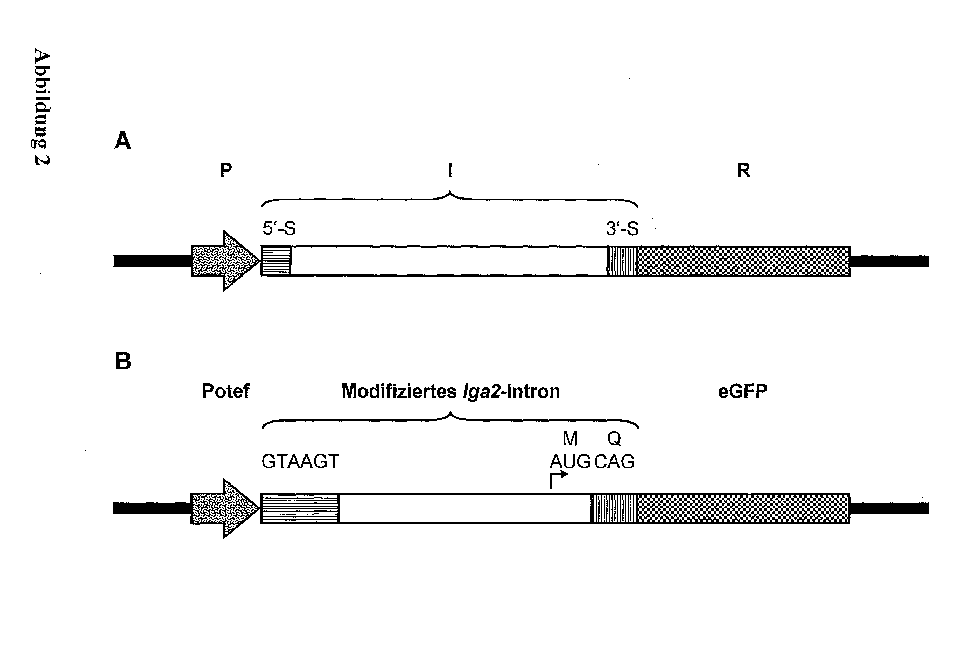

In Abb. 2 ist ein möglicher Aufbau eines erfindungsgemäßen DNA-Konstruktes schematisch dargestellt. Abb. 2A zeigt dabei einen beispielhaften allgemeinen Aufbau eines solchen DNA-Konstruktes bestehend aus Promotor (P), Intron-Sequenz (I) und Reporter-Gen (R).

In einer bevorzugten Ausführungsform (vgl. Abb. 2B) wird die Expression des verwendeten DNA-Konstrukts durch den starken *otef*-Promotor (in Abb. 2B mit Potef bezeichnet) vermittelt (Spellig et al. (1996)). Alternativ kann der *oma*-Promotor verwendet werden (SEQ ID NO. 14). Als Test-Intron im DNA-Konstrukt wird das modifizierte endogene Intron Nr. 1 au dem *lga2*-Gen von *U. maydis* verwendet (Urban et al. (1996), SEQ ID NO. 10). Dabei wird die dritte Position der 5'-Splicing-Stelle verändert (G zu A) und so dem vorherrschenden Konsensus angepasst. Sie besitzt in diesem Fall die Sequenz 5'-GTAAGT-3'. Die 3'-Splicing-Stelle besitzt die Sequenz CAG (codierend für die Aminosäure Glutamin (Q)), direkt davor befindet sich das Startcodon AUG (codierend für die Aminosäure Methionin (M)). Aus dieser Anordnung resultiert ein artifizielles Intron, das weder ein Start- noch ein Stoppcodon im entsprechenden Leserahmen besitzt. Als Reporter-Gen dient das eGFP-Allel (Fa. Clontech).

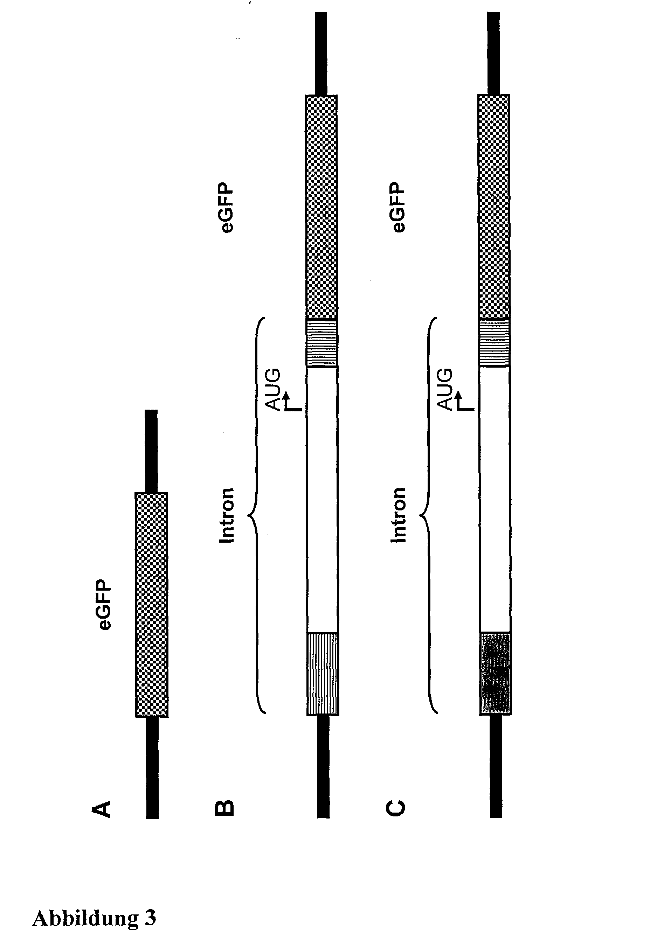

Der Aufbau der DNA-Konstrukte erlaubt nach Einbringen in eine Wirtszelle oder einen Wirtsorganismus bei Anwendung in einem Assay die Unterscheidung von Zellen, in denen Splicing stattfindet gegenüber solchen, in denen die Splicing-Funktion inhibiert ist. Die Resultate der einzelnen Konstrukte auf mRNA-Ebene sind in Abb. 3 dargestellt. Unter Wachstumsbedingungen (z.B. in Standard-Nährmedien wie PD-Medium, PD = Potato Dextrose, oder in geeigneten Minimalmedien) wird Splicing nicht inhibiert und Intronsequenzen werden entfernt. Das führt dazu, dass das Startkodon (AUG) im DNA-Konstrukt herausgespleißt wird. Die transkribierte GFP-mRNA enthält kein korrektes Translationsinitiationssignal und somit kann keine Reporter-Gen-Aktivität nachgewiesen werden (siehe Abb. 3A).

Falls jedoch ein Inhibitor Splicing verhindert, bleibt das Intron und somit auch das AUG-Startkodon erhalten (siehe Abb. 3B). Am Startkodon des GFP-Gens kann die Translation beginnen und das GFP-Protein wird exprimiert.

Da bislang kein Inhibitor für mRNA-Splicing zur Verfügung steht, wurde, um diese Situation zu simulieren (Positivkontrolle), ein Plasmid hergestellt, indem die 5'-Splicing-Stelle an einer Base mutiert ist (siehe Abb. 3C). Dadurch wird die Expression von GFP auch dann ermöglicht, wenn keine Inhibition erfolgt, da nun das Startkodon nicht mehr herausgspleißt werden kann. Des Weiteren kann dieses Konstrukt verwendet werden, um die maximal mögliche GFP-Fluoreszenz im Falle einer Inhibition zu ermitteln.

Die einzelnen Fälle (entsprechend Abb. 3) dieser besonders bevorzugten Ausführungsform lassen sich wie folgt zusammenfassen:

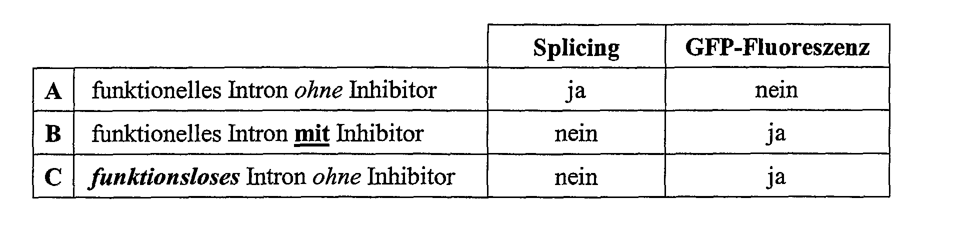

Die vorliegende Erfindung betrifft weiterhin ein Verfahren zum Herstellen der erfindungsgemäßen DNA-Konstrukte, das dadurch gekennzeichnet ist, dass man
a) in einem ersten Schritt
   i) ein geeignetes Intron aus einem geeigneten Gen der genomischen DNA einer geeigneten Wirtszelle bzw. einem geeigneten Wirtsorganismus mit Hilfe der Primer I-5' (für die 5'-Flanke des Introns) und I-3' (für die 3'-Flanke des Introns) amplifiziert, wobei die Sequenz des Introns gegebenenfalls durch die Wahl eines geeigneten Primers gezielt modifiziert wird,
   ii) ein geeignetes Reporter-Gen aus einer geeignete Quelle, z.B. einem Plasmid, mit den Primern RG-5' und RG-3' (für die beiden Flanken des Reporter-Gens) amplifiziert,
b) in einem zweiten Schritt die beiden Amplifikate aus Schritt (a) unabhängig voneinander in ein geeignetes Plasmid I kloniert, wodurch man die beiden Plasmide II (Intron) und III (Reporter-Gen) erhält,
c) in einem dritten Schritt
   i) aus dem Plasmid II (Intron) mit Restriktionsenzymen, die die Schnittstellen R1 und R2 erzeugen, das Intronfragment herausschneidet,
   ii) aus dem Plasmid III (Reporter-Gen) mit Restriktionsenzymen, die die Schnittstellen R2 und R3 erzeugen, das Reporter-Gen-Fragment herausschneidet,
   iii) einen geeigneten Vektor, der einen geeigneten Promotor enthält, z.B. ein Plasmid IV, das zumindest zwei unterschiedliche Restriktionsschnittstellen R1 und R3 besitzt, enzymatisch so restringiert, dass man die Schnittstellen R1 und R3 erhält, und
   iv) die drei erhaltenen Fragmente so ligiert, dass man ein Plasmid V erhält, indem eine Intron-Sequenz und das Reporter-Gen funktionell verknüpft sind.

Geeignete Introns, Wirtszellen bzw. -organismen und Reporter-Gene, die in Schritt (a) des erfindungsgemäßen Verfahrens zum Herstellen der DNA-Konstrukte benötigt werden, wurden bereits oben in Zusammenhang mit dem erfindungsgemäßen Verfahren zum Nachweis der Funktionsfähigkeit des Splicing beschrieben.

Bevorzugt setzt man Introns und Reporter-Gene ein, deren DNA-Sequenzen bereits bekannt sind oder durch Sequenzierung zugänglich wurden, so dass sich geeignete Primer zur Amplifikation herstellen lassen.

Genomische DNA lässt sich nach molekularbiologischen Standard-Methoden isolieren. Für die Isolierung der genomischen DNA aus *U. maydis* siehe Hoffinann and Winston (1987).

In Schritt (a i) wird gegebenenfalls die Sequenz des Introns modifiziert. Z.B. kann die 5'-Splicingsite durch Mutation des dritten Nucleotids von G (Guanin) nach A (Adenin) der vorherrschenden für das Splicing wichtigen Konsensussequenz angepasst werden. Diese Punktmutation kann auf dem Fachmann bekannte Weise durch geeignete, z.B. synthetisch hergestellte Primer bei der PCR-Reaktion eingeführt werden. Man erhält ein modifiziertes, funktionelles Intron.

Ein geeignetes Plasmid I, welches in Schritt (b) verwendet wird, ist dadurch gekennzeichnet, dass es unterschiedliche Schnittstellen für wenigstens drei Restriktionsenzyme besitzt, wodurch sich zumindest drei unterschiedliche Restriktionsschnittstellen R1, R2 und R3 erzeugen lassen. Als geeignetes Plasmid I sei z.B. das Plasmid pCRIITopo (Fa. Invitrogen) genannt. Die Klonierung in dieses Plasmid erfolgt in der Weise, dass das Plasmid II, das hinterher die Intron-Sequenz enthält, mit einer anderen Kombination an Restriktionsenzymen geschnitten werden kann als das Plasmid III, das das Reporter-Gen enthält, wobei die Schnittstellen so gewählt werden, dass beide Fragmente an einem Ende gleich sind und in Schritt (c) ligiert werden können.

In Schritt (c) werden zunächst aus drei verschiedenen Plasmiden Fragmente in der Weise herausgeschnitten, dass sich jeweils zwei Enden ligieren lassen.

Der in Schritt (c iii) verwendete Vektor (bzw. das Plasmid IV) wird so ausgewählt bzw. konstruiert, dass er bereits einen geeigneten Promotor enthält (z.B. pCA123).

Das erfindungsgemäße Verfahren zum Herstellen der DNA-Konstrukte kann sowohl dazu verwendet werden, geeignete Konstrukte für das Verfahren zum Nachweis der Funktionsfähigkeit des Splicing herzustellen, als auch dazu, Konstrukte mit bestimmten Eigenschaften (z.B. mit einer Mutation in der 5'-Splicingsite der Intron-Sequenz, vgl. oben und Abb. 3C) zu erzeugen.

Das am Ende von Schritt (c) erhaltene Plasmid V kann schließlich dazu verwendet werden, das erfindungsgemäße DNA-Konstrukt in eine Wirtszelle bzw. einen Wirtsorganismus einzubringen (Schritt (B) des erfindungsgemäßen Verfahrens).

Beim Herstellen eines DNA-Konstruktes geht man in einer bevorzugten Ausführungsform beispielsweise wie folgt vor.
a) In einem ersten Schritt
   i) wird z.B. das erste Intron des *lga2*-Gens aus der genomischen DNA des *U. maydis*-Stammes Um518 mit Hilfe der Primer I-5' (für die 5'-Flanke des Introns, z.B. der Primer lga25', SEQ ID NO. 1, durch den die 5'-Splicingsite im *lga2*-Intron von G zu A im Vergleich zur Wildtypsequenz mutiert wird) und I-3' (für die 3'-Flanke des Introns, z.B. der Primer CA52, SEQ ID NO. 2) amplifiziert, und
   ii) z.B. das *egfp*-Gen als Reporter-Gen aus dem Plasmid pCA123 (bestehend aus dem *otef*-Promotor, dem egfp-Gen, einem pSP72 Backbone und der cbx-Resistenz) mit den Primern RG-5' (z.B. der Primer CA53, SEQ ID NO. 3) und RG-3' (z.B. der Primer 3'GFP-Not, SEQ ID NO. 4) amplifiziert.
b) In einem zweiten Schritt werden die beiden Amplifikate aus Schritt (a) getrennt voneinander in ein geeignetes Plasmid I (z.B. das Plasmid pCRIITopo der Fa. Invitrogen) kloniert. Nach diesen Schritt erhält man einerseits das Plasmid II (z.B. das Plasmid pCRIITopo-lga2), welches die Intron-Sequenz enthält, und andererseits das Plasmid III (z.B. das Plasmid pCRIITopo-UeGFP), welches das Reporter-Gen enthält.
c) In einem dritten Schritt
   i) wird aus dem Plasmid II (pCRIITopo-lga2) das *lga2*-Intron als 74 bp *Bgl*II/*Sph*I-Fragment herausgeschnitten,
   ii) aus dem Plasmid III (pCRIITopo-UeGFP) das *egfp*-Gen als 726 bp *Sph*I/*Not*I-Fragment herausgeschnitten,
   iii) der Vektor (z.B. das Plasmid pCA123, enthaltend den *otef*-Promotor) mit den Restriktionsenzymen *Bam*HI und *Not*I restringiert und
   iv) die drei erhaltenen Fragmente zusammen ligiert, wodurch man ein Plasmid V (p123-lga2-eGFP) erhält.

Um das DNA-Konstrukt zu erhalten, das an der 5'-Splicingsite der Intron-Sequenz in der Weise mutiert ist, dass ein Splicing nicht mehr stattfinden kann, verwendet man in einer weiteren bevorzugten Ausführungsform in Schritt (a) den Primer lga25'mut (SEQ ID NO. 5), wodurch am Ende das Plasmid VI (p123-lga25'mut-eGFP) entsteht.

Die erfindungsgemäßen DNA-Konstrukte werden in Schritt (B) des erfindungsgemäßen Verfahrens in eine Wirtszelle oder einen Wirtsorganismus eingebracht.

Gegenstand der Erfindung sind demnach auch Wirtszellen und Wirtsorganismen, die die erfindungsgemäßen DNA-Konstrukte enthalten.

Geeignete Wirtszellen und -organismen wurden bereits oben im Zusammenhang mit der allgemeinen Beschreibung des erfindungsgemäßen Verfahrens genannt.

Im Schritt (B) des erfindungsgemäßen Verfahrens werden die erfindungsgemäßen DNA-Konstrukte in eine Wirtszelle oder einen Wirtsorganismus eingebracht. Allgemeine Verfahren zur Transformation, die hierzu verwendet werden können, wurden bereits oben beschrieben.

In eine bevorzugten Ausführungsform verwendet man ganze Organismen, besonders bevorzugt von *U. maydis*, ganz besonders bevorzugt den *U. maydis*-Stamm Um518.

Im allgemeinen geht man bei der Durchführung von Schritt (B) des erfindungsgemäßen Verfahrens beim Einbringen der DNA-Konstrukte in einen Wirt in der Weise vor, dass man die DNA-Konstrukte gegebenenfalls mit einem geeigneten Restriktionsenzym linearisiert und anschließend in den Wirt einbringt.

Zur Linearisierung der DNA verwendet man bevorzugt solche Restriktionsenzyme, die die Integration der Konstrukte an bestimmten Loci des Wirtsorganismus bevorzugt eintreten lassen.

In einer bevorzugten Ausführungsform geht man beispielsweise so vor, dass man das Plasmid V (p123-lga2-eGFP) mit dem Restriktionsenzym *Ssp*I linearisiert und über die PEG/Protoplastenmethode in das Genom des haploiden *U. maydis* Stammes Um518 transformiert (vgl. Schulz et al. (1990)). Durch den Schnitt mit *Ssp*I erfolgt die Integration der Konstrukte präferentiell am *cbx*-Locus von *U. maydis*. Durch das Restriktionenzym *Ssp*I wird hier das *cbx*-Resistenzgen im offenen Leserahmen geschnitten. Die Resistenz gegenüber Carboxin wird durch eine Punktmutation in der Eisen-Schwefel-Untereinheit der endogenen Succinat-Dehydrogenase *ip*^{*s*} vermittelt. Durch homologe Rekombination wird nun das Konstrukt so integriert, dass es von der wt-Kopie auf der einen Seite und dem resistenzverleihenden Gen auf der anderen Seite flankiert wird. Ektopische Integrationen sind möglich, wenn das Konstrukt z.B. während der Transformation rezirkularisiert oder Teile integrieren, die nicht vollständig geschnitten waren. Mit dem Plasmid VI (p123-lga25'mut-eGFP) verfährt man in derselben Weise.

Um den Integrationsort der Splicing-Konstrukte zu bestimmen, wird zunächst genomische DNA des Wirts isoliert (vgl. Hoffmann and Winston (1987)) und anschließend mit bestimmten Restriktionsenzymen verdaut (z.B. *Hind*III und *Bam*HI). Beim Nachweis verwendet man im Fall der Integration in den *cbx*-Locus bei *Ustilago maydis* als Sonde ein PCR-Fragment aus dem Plasmid pCBX122 (Keon et al. (1991)).

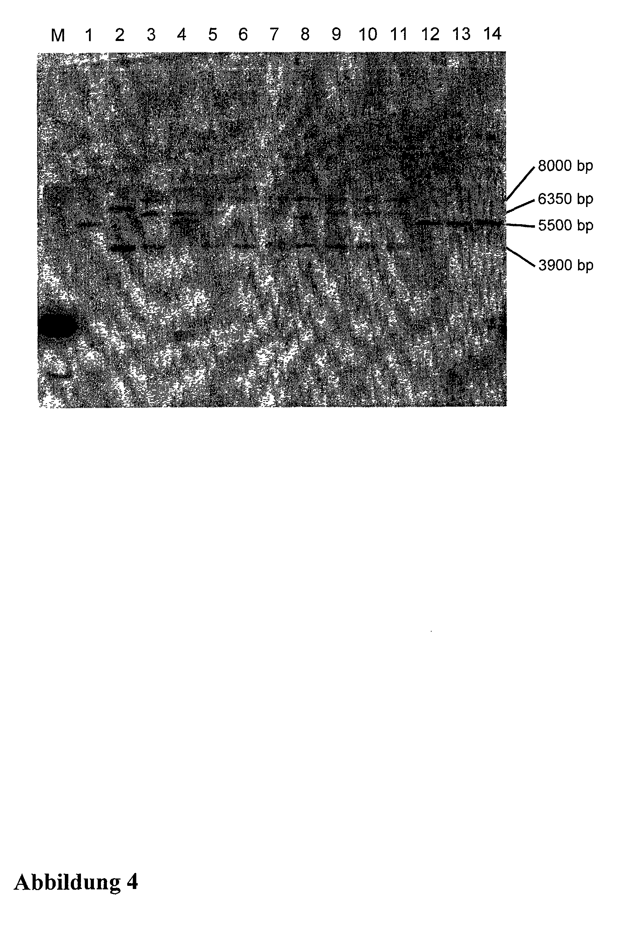

Durch die Integration der Konstrukte am immer gleichen Gen-Ort wird eine vergleichbare Expression in den unterschiedlichen Stämmen ermöglicht. Die Einzelintegrationsereignisse werden mittels PCR und durch Southernblot-Analyse bestätigt (siehe Abb. 4). Die Stämme DS#873 und DS#877 (Spur4 bzw. Spur8, Abb. 4) tragen jeweils zwei Kopien der Konstrukte p123-lga2-eGFP und p123-lga25'mut-eGFP.

Die Kontrolle der erfolgreichen Transformation erfolgt nach Anzucht der Kolonien (z.B. von *U. maydis* auf PD-Platten) durch Anregung der eGFP-Fluoreszenz durch eine starke Lichtquelle mit Licht der Wellenlänge 485 nm (10 nm Bandbreite). Die Fluoreszenz des eGFP-Proteins wird anschließend durch Anwendung eines Filters mit einer Durchlässigkeit bei 510 nm (10 nm Bandbreite) sichtbar gemacht.

Als Ergebnis zeigt sich, dass von 5 Transformanden, die das Konstrukt mit einer 5'mutierten Splicesite trugen, alle grüne Fluoreszenz emittierten. Die 5'-Splicesite des *lga2*-Introns wird durch eine Punktmutation unter Verwendung der Primer CA52 (SEQ ID NO. 2) und lga25'mut (SEQ ID NO. 5) wie oben beschrieben verändert, wodurch man ein Intron mit der Sequenz entsprechend der SEQ ID NO. 11 erhält. Der Stamm DS#877 enthält ein entsprechendes Konstrukt. Diese Punktmutation in der 5'-Splicesite ist demnach ausreichend, das Splicing des Introns zu verhindern. Im Gegensatz dazu konnte in keinem der Stämme, die ein wt-lga2-Intron trugen, GFP-Fluoreszenz nachgewiesen werden. Mit dem modifizierten wt-Intron (vgl. die Sequenz mit der SEQ ID NO. 10) ist Splicing möglich und entsprechend ist keine GFP-Fluoreszenz nachweisbar.

Eine weitere Möglichkeit, Splicing nachzuweisen, besteht auf molekularer Ebene durch die Anwendung von RT-PCR-Experimenten (Reverse Transcription Polymerase Chain Reaction).

Im allgemeinen geht man bei diesen RT-PCR-Experimenten in der Weise vor, dass man zunächst Gesamt-RNA isoliert (vgl. Schmitt et al. (1990)). Anschließend wird daraus polyA⁺-RNA präpariert und durch RT-PCR amplifiziert. Zur Bestimmung der exakten Länge der einzelnen PCR-Produkte werden diese sequenziert. Durch die Auswahl geeigneter Primer-Kombinationen kann direkt festgestellt werden, ob das primäre Genprodukt des Reporter-Gens vorhanden ist (jeweils Primer für das 5'- und 3'-Ende dieses Gens), welches das Intron enthält (Primer für das 5'-Ende des Introns und 3'-Ende des Reporter-Gens). Durch eine dritte Primer-Kombination (Primer für die 5'UTR-Region und das 3'-Ende des Reporter-Gens) kann im Vergleich gespleißte von ungespleißter RNA unterschieden werden, da unterschiedlich lange Fragmente entstehen.

In einer bevorzugten Ausführungsform geht man so vor, dass man die *U. maydis*-Stämme DS#873 und DS#877 für RT-PCR-Experimente zum Nachweis der GFP-mRNA einsetzt. Als Positiv-Kontrolle für die RT-PCR verwendet man den *U. maydis-Stamm* UMA3, in dessen *cbx*-Locus der Vektor pCA123, der das eGFP-Gen unter der Kontrolle des *otef*-Promotors trägt, integriert wurde und somit das *egfp*-Gen konstitutiv exprimiert.

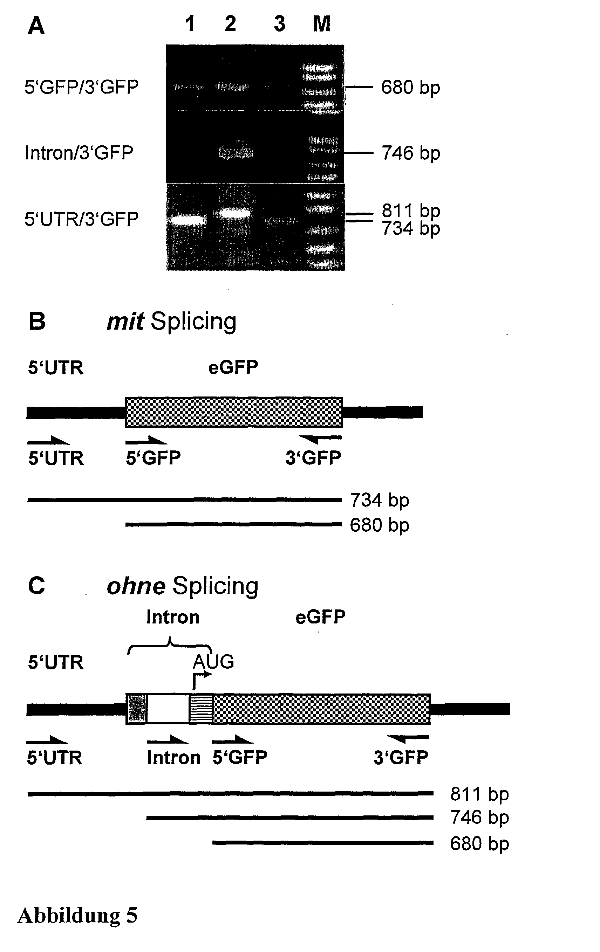

Um GFP-Expression unabhängig von der mRNA-Spezies nachzuweisen, wurde zunächst eine cDNA mit den Primern 5'GFP (SEQ ID NO. 6) und 3'GFP (SEQ ID NO. 7) gewählt. Ist eine entsprechende GFP-mRNA vorhanden, so kann ein 680 bp langes Fragment amplifiziert werden. Es zeigte sich, dass in allen untersuchten Stämmen nur dieses Fragment amplifiziert werden konnte (Abb. 5A, oberes Drittel, Spuren 1 bis 3), also ein GFP-Transkript vorlag.

Um Stämme zu identifizieren, bei denen das Intron nicht gespleißt wurde, wurde eine Primerkombination (Intron/3'GFP, SEQ ID NO. 9/SEQ ID NO. 7) gewählt, die nur bei vorhandenem Intron ein Ergebnis liefert. Hierbei zeigt nur der Stamm DS#877 ein positives Resultat in Form eines 746 bp langen mRNA-Fragments, da dieser Stamm das 5'-mutierte Intron trägt (vgl. Abb. 5A, mittleres Drittel, Spur 2).

Zur direkten Unterscheidung von gespleißter und ungespleißter mRNA verwendet man die Primerkombination 5'UTR/3'GFP (SEQ ID NO. 8/SEQ ID NO. 7). Dabei werden abhängig vom Splicing unterschiedliche lange Amplikons generiert. Findet Splicing statt, ist die GFP-mRNA 734 bp lang, findet kein Splicing statt ist sie jedoch 811 bp lang, da das Intron noch vorhanden ist. Die Analyse zeigte ein nur 734 bp langes PCR-Fragment für die Stämme UMA3 und DS#873 (Abb. 5A, unteres Drittel, Spuren 3 bzw. 1) bzw. ein 811 bp langes Amplikon im Stamm DS#877 (Abb. 5A, Spur 2). Dadurch ist gezeigt, dass der Unterschied in den Transkriptlängen auf Splicing zurückzuführen ist.

Schematisch ist das Resultat dieser Untersuchungen in Abb. 5B (Splicing findet statt) und Abb. 5C (kein Splicing) dargestellt. Dieses Ergebnis lässt sich leicht auf den Fall anderer Reporter-Gene, Introns und Promotoren übertragen.

Gegenstand der vorliegenden Erfindung sind auch Verfahren zum Auffinden von chemischen Verbindungen, die auf das Spliceosom und/oder einen seiner Bestandteile in einer Weise einwirken, die zur Modulation des Splicing, bevorzugt zur Inhibition, führt.

Gegenstand der vorliegenden Erfindung sind auch Verfahren zum Auffinden von chemischen Verbindungen, die auf das Assembly des Spliceosoms und/oder einer der daran beteiligten Komponenten in einer Weise einwirken, die zur Modulation des Splicing, bevorzugt zur Inhibition, führt.
Gegenstand der vorliegenden Erfindung ist also ein Verfahren zum Identifizieren von Inhibitoren des Splicing, das dadurch gekennzeichnet ist, dass man
(a) ein erfindungsgemäßes DNA-Konstrukt (siehe oben) herstellt;
(b) dieses DNA-Konstrukt aus Schritt (a) in eine erfindungsgemäße Wirtszelle oder einen erfindungsgemäßen Wirtsorganismus (siehe oben) einbringt;
(c) diese Wirtszelle oder den Wirtsorganismus aus Schritt (b) mit einer Einzelsubstanz oder einer Mischung von mehreren chemischen Verbindungen in Kontakt bringt,
(d) die Anwesenheit oder Abwesenheit des Reporter-Gen-Produktes in Gegenwart der Einzelsubstanz oder einer Mischung von mehreren chemischen Verbindungen vergleicht mit der Anwesenheit oder Abwesenheit des Reporter-Gen-Produktes bei deren Abwesenheit, und
(e) gegebenenfalls die Verbindung oder Verbindungen identifiziert, wodurch die Funktionalität des Splicing beeinflusst wird.

Bevorzugt ist ein Verfahren zum Identifizieren von Inhibitoren des Splicing, das dadurch gekennzeichnet ist, dass man
(a) ein DNA-Konstrukt herstellt, in dem das Reporter-Gen in der Weise mit dem Intron verknüpft ist, dass die Entstehung des Reporter-Gen-Produktes bei gestörtem bzw. ganz unterdrücktem Splicing gewährleistet ist.;
(b) das DNA-Konstrukt aus Schritt (a) in eine Wirtszelle oder einen Wirtsorganismus einbringt, bevorzugt in *Ustilago maydis*;
(c) die Wirtszelle oder den Wirtsorganismus aus Schritt (b) mit einer Einzelsubstanz oder einer Mischung von mehreren chemischen Verbindungen in Kontakt bringt,
(d) die Anwesenheit des Reporter-Gen-Produktes in Gegenwart der Einzelsubstanz oder einer Mischung von mehreren chemischen Verbindungen vergleicht mit der Anwesenheit des Reporter-Gen-Produktes bei deren Abwesenheit, und
(e) gegebenenfalls die Verbindung oder Verbindungen identifiziert, wodurch die Funktionalität des Splicing beeinflusst wird.

Gegenstand der vorliegenden Erfindung sind auch Verfahren zum Auffinden von chemischen Verbindungen, welche auf die Expression von Komponenten des Splicing-Apparates oder der für das Assembly notwendigen direkten Bausteine oder der jeweils zugehörigen Hilfskomponenten in einer Weise einwirken, die zur Modulation des Splicing, bevorzugt zur Inhibition, führt.

Gegenstand der vorliegenden Erfindung ist also auch ein Verfahren zum Identifizieren von Verbindungen, welche die Expression von Komponenten des Splicing-Apparates beeinflussen, das dadurch gekennzeichnet ist, dass man
(a) ein erfindungsgemäßes DNA-Konstrukt (siehe oben) herstellt;
(b) das DNA-Konstrukt aus Schritt (a) in eine erfindungsgemäße Wirtszelle oder einen erfindungsgemäßen Wirtsorganismus einbringt;
(c) die Wirtszelle oder den Wirtsorganismus aus Schritt (b) mit einer Einzelsubstanz oder einer Mischung von mehreren chemischen Verbindungen in Kontakt bringt,
(d) die Anwesenheit oder Abwesenheit des Reporter-Gen-Produktes in Gegenwart der Einzelsubstanz oder einer Mischung von mehreren chemischen Verbindungen vergleicht mit der Anwesenheit oder Abwesenheit des Reporter-Gen-Produktes bei deren Abwesenheit,
(e) die Polypeptid- und/oder RNA-Zusammensetzung des Spliceosoms bestimmt, und
(f) gegebenenfalls die Verbindung oder Verbindungen identifiziert, wodurch die Funktionalität des Splicing beeinflusst wird.

So stellen Modulatoren, die das Splicing beeinflussen, neue Wirkstoffe dar. Solche Wirkstoffe sind z.B. Fungizide, die in der Lage sind, die Lebensfähigkeit von Pilzen zu kontrollieren. Ebenfalls kommen Insektizide infrage, die den Befall mit unerwünschten Schädlingen kontrollieren, oder Herbizide, die für die Kontrolle des Wachstums von Unkräutern verwendet werden können. Ebenso gehören zu den erfindungsgemäß auffindbaren Wirkstoffen solche, die in Arzneimitteln z.B. zur Kontrolle von unerwünschtem Tumorwachstum verwendet werden können.

Im Falle von Fungiziden ist die Kontrolle insbesondere von phytopathogenen sowie für human- oder tierpathogenen Pilze, bei denen das Splicing ein Target für Antimycotica darstellt, von besonderem Interesse. Der Begriff *Fungizid* oder *fungizid,* wie er hierin verwendet wird, soll sich demgemäß auch auf den Begriff *Antimycotica* oder *antimycotisch* erstrecken.

Pilze aus folgenden Klassen seinen beispielhaft, d.h. nicht abschließend genannt: Plasmodiophoromycetes, Oomycetes, Chytridiomycetes, Zygomycetes, Ascomycetes, Basidiomycetes und Deuteromycetes, z. B.

Pythium-Arten, wie beispielsweise *Pythium ultimum,* Phytophthora-Arten, wie beispielsweise *Phytophthora infestans,* Pseudoperonospora-Arten, wie beispielsweise *Pseudoperonospora humuli* oder *Pseudoperonospora cubensis,* Plasmopara-Arten, wie beispielsweise *Plasmopara viticola,* Bremia-Arten, wie beispielsweise *Bremia lactucae,* Peronospora-Arten, wie beispielsweise *Peronospora pisi* oder *P. brassicae,* Erysiphe-Arten, wie beispielsweise *Erysiphe graminis,* Sphaerotheca-Arten, wie beispielsweise *Sphaerotheca fuliginea,* Podosphaera-Arten, wie beispielsweise *Podosphaera leucotricha,* Venturia-Arten, wie beispielsweise *Venturia inaequalis,* Pyrenophora-Arten, wie beispielsweise *Pyrenophora teres* oder *P. graminea* (Konidienform: Drechslera, Syn: Helminthosporium), Cochliobolus-Arten, wie beispielsweise *Cochliobolus sativus* (Konidienform: Drechslera, Syn: Helminthosporium), Uromyces-Arten, wie beispielsweise *Uromyces appendiculatus,* Puccinia-Arten, wie beispielsweise *Puccinia recondita,* Sclerotinia-Arten, wie beispielsweise *Sclerotinia sclerotiorum,* Tilletia-Arten, wie beispielsweise *Tilletia caries*; Ustilago-Arten, wie beispielsweise *Ustilago maydis, Ustilago nuda* oder *Ustilago avenae,* Pellicularia-Arten, wie beispielsweise *Pellicularia sasakii*, Pyricularia-Arten, wie beispielsweise *Pyricularia oryzae*, Fusarium-Arten, wie beispielsweise *Fusarium culmorum*, Botrytis-Arten, wie beispielsweise *Botrytis cinerea*, Septoria-Arten, wie beispielsweise *Septoria nodorum*, Leptosphaeria-Arten, wie beispielsweise *Leptosphaeria nodorum*, Cercospora-Arten, wie beispielsweise *Cercospora canescens*, Alternaria-Arten, wie beispielsweise *Alternaria brassicae* oder Pseudocercosporella-Arten, wie beispielsweise *Pseudocercosporella herpotrichoides.*

Von besonderem Interesse sind z. B. auch *Saccharomyces cerevisiae, Magnaporthe grisea, Aspergillus nidulans, Cochliobulus heterostrophus, Nectria hematococca, Botrytis cinerea, Geaumannomyces sp., Pichia pastoris, Ustilago maydis* und Phytophtora Spezies.

Humanpathogene Pilze von besonderem Interesse sind im Folgenden aufgeführt zusammen mit den Krankheitsbildern, die sie hervorrufen können:

Dermatophyten, wie z.B. *Trichophyton spec., Microsporum spec., Epidermophyton* *floccosum* oder *Keratomyces ajelloi,* die z.B. Fußmykosen (Tinea pedis) hervorrufen, Hefen, wie z.B. *Candida albicans*, der z.B. Soor-Ösophagitis und Dermatitis hervorruft, *Candida glabrata, Candida krusei* oder *Cryptococcus neoformans*, die z.B. pulmonale Cryptococcose und Torulose hervorrufen können,

Schimmelpilze, wie z.B. *Aspergillus fumigatus, A. flavus, A. niger,* die z.B. bronchopulmonale Aspergillose oder Pilzsepsis hervorrufen, *Mucor spec., Absidia spec.* oder *Rhizopus spec.,* die z.B. Zygomykosen (intravasale Mykosen) hervorrufen, *Rhinosporidium seeberi,* der z.B. chronische granulomatöse Pharyngitis und Tracheitis hervorruft, *Madurella myzetomatis,* der z.B. subkutane Myzetome hervorruft, *Histoplasma capsulatum,* der z.B. retikuloendotheliale Zytomykose und M. Darling hervorruft, *Coccidioides immitis,* der z.B. pulmonale Coccidioidomykose und Sepsis hervorruft, *Paracoccidioides brasiliensis,* der z.B. brasilianische Blastomykose hervorruft, *Blastomyces dermatitidis,* der z.B. Gilchrist-Krankheit und nordamerikanische Blastomykose hervorruft, *Loboa loboi,* der z.B. Keloid-Blastomykose und Lobo's Krankheit hervorruft, und *Sporothrix schenckii*, der z.B. Sporotrichose (granulomatöse Hautmykose) hervorruft.

Modulatoren können Antagonisten bzw. Inhibitoren sein. Von besonderem Interesse sind im Falle des Splicing Inhibitoren, die durch das Ausschalten des Splicing-Vorgangs zur entsprechenden Wirkung oben genannter möglicher Wirkstoffe führen.

Gegenstand der vorliegenden Erfindung ist daher insbesondere auch die Verwendung des Spliceosoms als Angriffspunkt für Wirkstoffe und dessen Verwendung in Verfahren zum Auffinden von Modulatoren des Splicing.

Gegenstand der vorliegenden Erfindung ist deshalb auch die Verwendung von DNA-Konstrukten, die die Aktivität des Splicing direkt oder indirekt anzeigen, und von Wirtszellen oder Wirtsorganismen, die diese enthalten, zum Auffinden von Modulatoren des Splicing.

Der Ausdruck "Modulator", wie er hierin verwendet wird, stellt den Oberbegriff zu Agonist und Antagonist bzw. Aktivator und Inhibitor dar. Der Ausdruck "Agonist" bzw. "Aktivtor", bezieht sich dabei auf ein Molekül, das die Aktivität des Splicing beschleunigt oder verstärkt; der Ausdruck "Antagonist" bzw. "Inhibitor" auf ein Molekül, das die Aktivität des Splicing verlangsamt oder verhindert.

Modulatoren können kleine organisch-chemische Moleküle, Peptide oder Antikörper sein, die an das Spliceosom und/oder einen seiner Bestandteile selbst binden, das Assembly des Spliceosoms und/oder einer der daran beteiligten Komponenten beeinflussen, oder auf die Expression von Komponenten des Splicing-Apparates oder der für das Assembly notwendigen direkten Bausteine oder der jeweils zugehörigen Hilfskomponenten einwirken. Modulatoren können alle solchen kleinen organischchemischen Moleküle, Peptide oder Antikörper sein, die eine orts- und/oder zeitgerechte Durchführung des Splicing beeinflussen.

Vorzugsweise handelt es sich bei den Modulatoren um kleine organisch-chemische Verbindungen.

Die Modulatoren können durch ihre Einwirkung auf das Splicing die zellulären Vorgänge auf eine Weise verändern, die zur Apathogenität und/oder zum Absterben von damit behandelten Pilzen führt.

Die Modulatoren können durch ihre Einwirkung auf das Splicing die zellulären Vorgänge auf eine Weise verändern, die zum Absterben von damit behandelten Schädlingen führt.

Die Modulatoren können durch ihre Einwirkung auf das Splicing die zellulären Vorgänge auf eine Weise verändern, die zum Absterben von damit behandelten Unkräutern führt.

Die Modulatoren können durch ihre Einwirkung auf das Splicing die zellulären Vorgänge auf eine Weise verändern, die zum Absterben von damit behandelten Tumoren führt.

Gegenstand der vorliegenden Erfindung sind damit auch Modulatoren, vorzugsweise Inhibitoren des Splicing, die mit Hilfe eines der vorstehend oder nachfolgend beschriebenen Verfahren zum Identifizieren von Modulatoren des Splicing gefunden wurden.

Bislang ist nicht bekannt geworden, dass Splicing bei phytopathogenen (pflanzenpathogenen) Pilzen ein hervorragendes Target für Fungizide darstellt und dass mit Hilfe des Splicing Verbindungen gefunden werden können, die als Fungizide eingesetzt werden können. In der vorliegenden Anmeldung wird diese Möglichkeit zum ersten Mal beschrieben und belegt. Weiter werden die notwendigen Hilfsmittel zum Nachweis der Funktionsfähigkeit des Splicing, wie DNA-Konstrukte und Verfahren zu deren Herstellung zur Verfügung gestellt.

Gegenstand der Erfindung ist daher die Verwendung von Splicing-Modulatoren als Fungizide und/oder Antimycotica.

Bislang ist auch nicht bekannt geworden, dass Splicing bei tierischen Schädlingen ein hervorragendes Target für Insektizide darstellt und dass mit Hilfe des Splicing Verbindungen gefunden werden können, die als Insektizide eingesetzt werden können. In der vorliegenden Anmeldung wird diese Möglichkeit zum ersten Mal genannt. Wieter werden die notwendigen Hilfsmittel zum Nachweis der Funktionsfähigkeit des Splicing, wie DNA-Konstrukte und Verfahren zu deren Herstellung zur Verfügung gestellt.

Gegenstand der Erfindung ist daher weiterhin die Verwendung von Splicing-Modulatoren als Insektizide.

Bislang ist auch nicht bekannt geworden, dass Splicing bei Unkräutern ein hervorragendes Target für Herbizide darstellt und dass mit Hilfe des Splicing Verbindungen gefunden werden können, die als Herbizide eingesetzt werden können. In der vorliegenden Anmeldung wird diese Möglichkeit zum ersten Mal genannt. Weiter werden die notwendigen Hilfsmittel zum Nachweis der Funktionsfähigkeit des Splicing, wie DNA-Konstrukte und Verfahren zu deren Herstellung zur Verfügung gestellt.

Gegenstand der Erfindung ist daher weiterhin die Verwendung von Splicing-Modulatoren als Herbizide.

Weiterhin umfasst die vorliegende Erfindung Verfahren zum Auffinden von chemischen Verbindungen, welche die Expression von Bestandteilen des Spliceosoms oder von Komponenten, die für das Assembly des Spliceosoms notwendig sind, verändern. Auch solche "Expressionsmodulatoren" können neue fungizide Wirkstoffe darstellen. Expressionsmodulatoren können kleine organisch-chemische Moleküle, Peptide oder Antikörper sein, die an die regulatorischen Regionen der für die erfindungsgemäßen Polypeptide codierenden Nukleinsäuren binden. Weiterhin können Expressionsmodulatoren kleine organisch-chemische Moleküle, Peptide oder Antikörper sein, die an ein Molekül binden, welches wiederum an regulatorische Regionen der für die Bestandteile des Spliceosoms oder von Komponenten, die für das Assembly des Spliceosoms notwendig sind, codierenden Nukleinsäuren bindet, und dadurch deren Expression beeinflusst. Expressionsmodulatoren können auch Antisense-Moleküle sein.

Gegenstand der vorliegenden Erfindung sind ebenfalls Expressionsmodulatoren des Splicing, die mit Hilfe eines nachstehend beschriebenen Verfahrens zum Identifizieren von Expressionsmodulatoren gefunden werden.

Gegenstand der Erfindung ist auch die Verwendung von Expressionsmodulatoren als Fungizide und/oder Antimycotica.

Gegenstand der Erfindung ist auch die Verwendung von Expressionsmodulatoren als Insektizide.

Gegenstand der Erfindung ist auch die Verwendung von Expressionsmodulatoren als Herbizide.

Gegenstand der Erfindung ist auch die Verwendung von Expressionsmodulatoren als Tumorstatika.

Die erfindungsgemäßen Verfahren schließen HTS und UHTS ein. Dafür können sowohl Wirtszellen als auch Wirtsorganismen verwendet werden, die die erfindungsgemäßen DNA-Konstrukte enthalten.

Um Modulatoren der erfindungsgemäßen Polypeptide aufzufinden, können Wirtszellen oder Wirtsorganismen, die ein erfindungsgemäßes DNA-Konstrukt enthalten, zusammen mit einer Einzelsubstanz oder einer Mischung aus mehreren Substanzen, die jeweils als Wirkstoffkandidaten infrage kommen, inkubiert werden. Die Fähigkeit eines Wirkstoffkandidaten, die Aktivität des Splicing zu inhibieren, wird durch das im DNA-Konstrukt verwendete Reporter-Gen dadurch angezeigt, dass entweder das Genprodukt selbst oder die Aktivität des Genproduktes einen messbaren Effekt zeigt.

Mischungen von potenziellen Wirkstoffkandidaten können beispielsweise aus 2, 10, 50, 100 oder 1000 verschiedenen Verbindungen bestehen. Es sind aber auch beliebige andere Mischungen möglich.

Verwendet man bei dem Verfahren zum Auffinden von Modulatoren des Splicing Mischungen von Wirkstoffkandidaten, muss bei einem Positivergebnis eine Dekonvolution erfolgen, d.h. aus dem Gemisch muss die eigentlich wirksame Verbindung aufgefunden werden. Dies geschieht z.B. dadurch, dass man die ursprüngliche Mischung in Mischungen mit weniger Verbindungen oder zu Einzelsubstanzen aufteilt und das Verfahren entsprechend wiederholt.

Gegenstand der Erfindung ist auch ein Verfahren zum Auffinden von Splicing-Modulatoren, das dadurch gekennzeichnet ist, dass man eine Wirtszelle oder einen Wirtsorganismus, die/der das erfindungsgemäße DNA-Konstrukt enthält, mit einer Einzelsubstanz oder eine Mischung aus Substanzen, die alle als Modulator infrage kommen, in Kontakt bringt und anschließend das Genprodukt oder die Aktivität des Reporter-Gens nachweist oder gegebenenfalls quantifiziert.

Im allgemeinen geht man dabei so vor, dass man die infrage kommenden Testsubstanzen in einem geeigneten Lösungsmittel (z.B. Dimethylsulfoxid, Wasser oder Gemische aus beiden) löst. Zu dieser Lösung gibt man eine definierte Menge/Zahl an Wirtszellen bzw. -organismen und bestimmt nach festgelegten Zeitintervallen das Genprodukt oder die Aktivität des Reporter-Gens.

In einer bevorzugten Ausführungsform löst man die Testsubstanzen in Dimethylsulfoxid und legt ein Aliquot von 5 µL einer 100 µM-Lösung in einem Inkubationsgefäß vor. Danach gibt man 45 µL Zellen (bevorzugt von *U. maydis*), die das oben beschriebene DNA-Konstrukt mit dem *egfp*-Gen als Reporter-Gen enthalten und zuvor in einem Minimalmedium bis zu einer OD₆₀₀ von 1,25 gewachsen waren, zu. Nach 0 h, 3 h und 6 h wird die Fluoreszenz gemessen. Die Differenzen Δ3 h-0 h und Δ6 h-3 h im Vergleich zu den Kontrollen stellen ein Maß für die Wirkung einer Substanz dar.

Als Kontrollen werden einerseits solche Stämme eingesetzt, die zwar das erfindungsgemäße DNA-Konstrukt enthalten, aber nicht in Kontakt mit potenziellen Wirkstoffsubstanzen gebracht wurden. Andererseits werden solche Stämme verwendet, die ein erfindungsgemäßes DNA-Konstrukt enthalten, das so mutiert ist, dass Splicing kontinuierlich unterdrückt ist. Dadurch ist im Verfahren das Reporter-Gen immer aktiv.

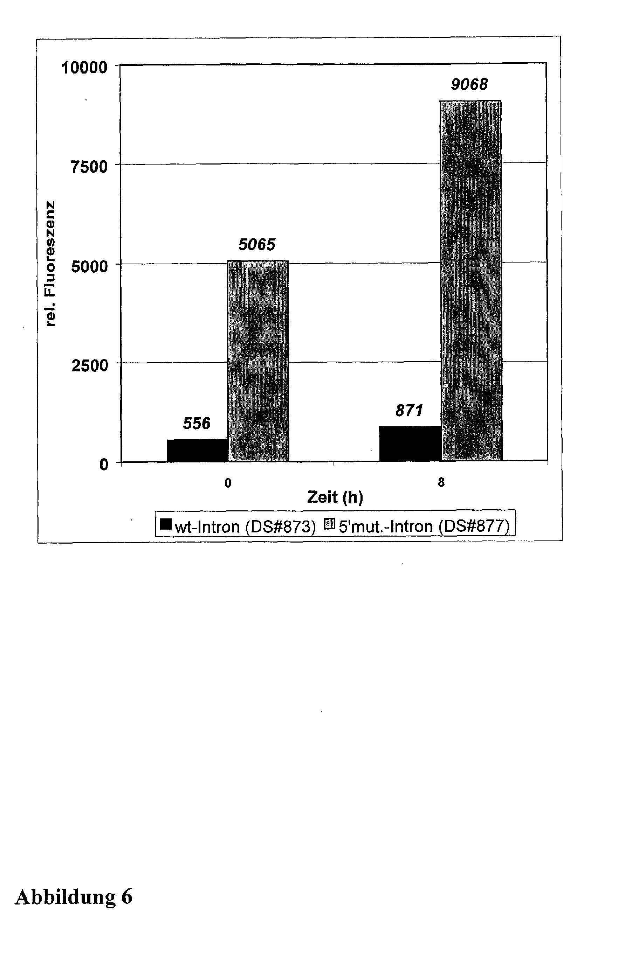

In einer bevorzugten Ausführungsform wird als Negativ-Kontrolle der Stamm DS#873 verwendet, der das modifizierte *lga2*-Intron enthält. Als Positiv-Kontrolle wird der Stamm DS#877 verwendet, der das *5'mut-lga2*-Intron enthält. Abb. 6 zeigt die zeitabhängige Zunahme der Fluoreszenz bei den beiden Kontrollen im Laufe von 8 h.

Das erfindungsgemäße Verfahren zum Auffinden von Modulatoren des Splicing-Vorganges kann auch im HTS und UHTS verwendet werden. Dazu erfolgt die Inkubation der Wirtszellen/-organismen mit den Testsubstanzen und die Messung der Fluoreszenz z.B. direkt in Mikrotiterplatten (MTP).

Unter Verwendung der oben genannten bevorzugten *U. maydis*-Stämme erhält man in einer MTP, bevorzugt einer mit 96 oder 384 Positionen, besonders bevorzugt einer mit 384 Positionen, die in der folgenden Tabelle dargestellten Mittelwerte an gemessener relativer Fluoreszenz.

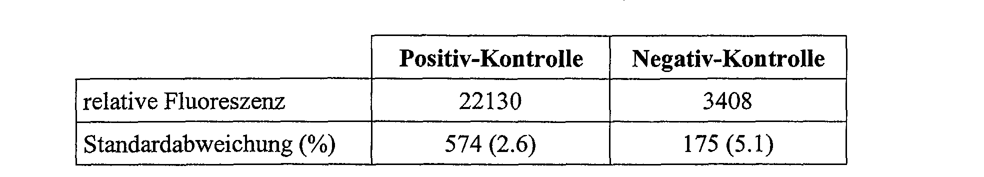

Der Wert der relativen Fluoreszenz der Positiv-Kontrolle, d.h. gleichzeitig der Maximalwert bei zu 100% verhindertem Splicing, war hierbei im Mittelwert um den Faktor 6,5 größer als der entsprechende Wert der Negativ-Kontrolle.

Gegenstand der vorliegenden Erfindung ist daher auch ein Verfahren zum Auffinden von Modulatoren des Splicing im HTS oder UHTS.

### Beispiele

### Molekularbiologische Standardmethoden

Molekularbiologische Standardmethoden (wie z.B. PCR, Ligation, Restriktion, Transformation von *E. coli,* RT-PCR, RNA-Isolierung, Southern-Analyse, Gelelektrophorese, DNA-Extraktion aus Gelen, Plasmid-Präparation), werden wie bei Sambrook et al. (1989) beschrieben durchgeführt.

### Herstellung der DNA-Konstrukte

a) In einem ersten Schritt
   i) wird das Intron Nr. 1 de *lga2*-Gens aus der genomischen DNA des *U. maydis-Stammes* Um518 mit Hilfe der Primer lga25' (SEQ ID NO. 1) und CA52 (SEQ ID NO. 2) amplifiziert, wodurch man das *lga2*-Intron als 74 bp-Fragment erhält, und
   ii) das *egfp*-Gen als Reporter-Gen aus dem Plasmid pCA123 mit den Primern CA53 (SEQ ID NO. 3) und 3'GFP-Not (SEQ ID NO. 4) amplifiziert, wodurch man das *egfp*-Gen als 726 bp-Fragment erhält.
b) In einem zweiten Schritt werden die beiden Amplifikate aus Schritt (a) getrennt voneinander in das Plasmid pCRIITopo (Fa. Invitrogen) kloniert. Dabei wird das dritte Nucleotid der 5'-Splicingsite im *lga2*-Intron von G zu A mutiert. Nach diesen Schritt erhält man einerseits das Plasmid pCRIITopo-lga2, welches die Intron-Sequenz enthält, und andererseits das Plasmid pCRIITopo-UeGFP, welches das Reporter-Gen enthält.
c) In einem dritten Schritt
   i) wird aus dem Plasmid pCRIITopo-lga2 das *lga2*-Intron als 78 bp *Bgl*II/*Sph*I-Fragment herausgeschnitten,
   ii) aus dem Plasmid pCRIITopo-UeGFP das *egfp*-Gen als 726 bp *Sph*I/*Not*I-Fragment herausgeschnitten,
   iii) das Plasmid pCA123 mit den Restriktionsenzymen *Bam*HI und *Not*I restringiert und
   iv) die drei erhaltenen Fragmente zusammen ligiert, wodurch man das Plasmid p123-lga2-eGFP erhält.

Um das DNA-Konstrukt zu erhalten, das an der 5'-Splicingsite der Intron-Sequenz in der Weise mutiert ist, dass ein Splicing nicht mehr stattfinden kann, verwendet man in Schritt (a) den Primer lga25'mut (SEQ ID NO. 5), wodurch am Ende das Plasmid p123-lga25'mut-eGFP entsteht.

### Herstellung des Plasmids pCA123

Aus dem Plasmid pOTEF-SG (Spellig et al. (1996)) wird der *otef*-Promotor als 890 bp langes *Pvu*II/*Nco*I-Fragment isoliert und in den *Pvu*II/*Nco*I geschnittenen Vektor pTEF-SG (Spellig et al. (1996)) ligiert. Im resultierenden Plasmid wird das SGFP Gen durch eine Restriktion mit *Nco*I/*Not*I herausgeschnitten und durch das *Nco*I/*Not*I geschnittene EGFP-Allel aus pEGFP-N1 (Fa. Clontech) ersetzt. Das resultierende Plasmid heißt pCA123. Es besteht aus einem pSP72 Backbone, dem *otef*-Promotor, dem eGFP-Gen (Clontech) und der cbx-Resistenzkassette.

### Einbringen der DNA-Konstrukte in U. maydis-Zellen

Das Einbringen der DNA-Konstrukte erfolgt in der Weise, dass man das Plasmid p123-lga2-eGFP mit dem Restriktionsenzym *Ssp*I linearisiert und über die PEG/-Protoplastenmethode in das Genom des haploiden *U. maydis* Stammes Um518 transformiert (vgl. Schulz et al. (1990)). Durch den Schnitt mit *Ssp*I erfolgt die Integration der Konstrukte präferentiell am *cbx*-Locus von *U. maydis.* Mit dem Plasmid p123-lga25'mut-eGFP verfährt man in derselben Weise.

Um den Integrationsort der Splicing-Konstrukte zu bestimmen, wird zunächst genomische DNA von *U. maydis* isoliert (vgl. Hoffmann and Winston (1987)) und anschließend mit den Restriktionsenzymen *Hind*III und *Bam*HI verdaut. Beim Nachweis verwendet man im Fall der Integration in den cbx-Locus bei *Ustilago maydis* als Sonde ein 283 bp PCR-Fragment aus dem Plasmid pCBX122, das die cbx-Resistenzkassette enthält. (Keon et al. (1991)). Der Nachweis erfolgte mit dem Dig-System (Fa. Roche). Die Amplifikation erfolgte mittels PCR und den Primern CBX-S3 (SEQ ID NO. 12) und CBX-A4 (SEQ ID NO. 13).

Durch die Integration der Konstrukte immer am gleichen Gen-Ort wird eine vergleichbare Expression in den unterschiedlichen Stämmen ermöglicht. Die Einzelintegrationsereignisse werden mittels PCR und durch Southernblot-Analyse bestätigt (siehe Abb. 4). Die Stämme DS#873 und DS#877 tragen jeweils zwei Kopien der Konstrukte p123-lga2-eGFP und p123-lga25'mut-eGFP.

### Nachweis der GFP-Expression

### Analyse der GFP-Fluoreszenz

Die *U. maydis* Reporterstämme werden bis zu einer optischen Dichte OD₆₀₀ von 0,8 in PD-Medium (Potato-Dextrose) bei 28°C inkubiert, durch Zentrifugieren geerntet (2200 g, Fa. Heraeus) und mit Minimalmedium (Holliday (1974)) in 0,1% Kelzan (Fa. Monsanto) auf die jeweils angegebene OD₆₀₀ gebracht. Anschließend werden die Zellen mit einem Multidrop (Fa. Labsystems) in 384 well MTP (Fa. Greiner, schwarz) transferiert. Die Messung erfolgt in einem Tecan ultra Fluoreszenz-Reader (Fa. Tecan) (Anregungswellenlänge 480 nm, 10 nm Bandbreite; Emissionswellenlänge 510 nm, 10 nm Bandbreite; Gain-Faktor 50; 3 Blitze).

### Nachweis der eGFP-Expression durch RT-PCR

Für RT-PCR Experimente wird Gesamt-RNA aus Flüssigkulturen von *U. maydis* isoliert (Schmitt et al. (1990)). Anschließend wird mit Hilfe von magnetischen PolydT-beads nach Angaben des Herstellers (Dynal) polyA⁺-RNA präpariert. Je 0,1-5 ng polyA⁺-RNA werden pro RT-PCR-Experiment eingesetzt, wobei für die Amplifikation und die Fluoreszenzmarkierung der Kit "SYBR green II" (Fa. Roche) verwendet wird. Für die Amplifikation wird eine "Light-cycler"-PCR-Maschine (Fa. Roche) eingesetzt. Die RT-PCR wurde nach Angaben des Herstellers (Roche) durchgeführt. Folgende Primerkombinationen wurden verwendet: GFP5' (SEQ ID NO. 6)/GFP3' (SEQ ID NO. 7); 5'UTR (SEQ ID NO. 8) / GFP3' (SEQ ID NO. 7) und Intron (SEQ ID NO. 9) / GFP3' (SEQ ID NO. 7). Die Amplicons wurden auf einem 1% Agarose-Gel aufgetrennt.

### Adaptierung der Teststämme auf ein 384-well-MTP-Format

Teststämme zur Identifizierung Splicing inhibierender Substanzen werden in PD-Medium inkubiert und bei einer OD₆₀₀ von 0,8 geerntet, in Wasser gewaschen und anschließend so in Minimalmedium aufgenommen, dass die OD₆₀₀ 2,5 beträgt. Je 50 µl der Kultur werden 1:1 in Minimalmedium mit 0,2% Kelzan (Fa. Monsanto) verdünnt, so dass eine OD₆₀₀ von 1,25 erreicht wird. Anschließend werden die Kulturen zu je 50 µl in die Kavitäten von MT-Platten pipettiert. Die Bestimmung der Fluoreszenz erfolgt wie oben beschrieben.

Um die zeitabhänige Zunahme der GFP Fluoreszenz in den Splicing Teststämmen zu analysieren, wird eine Kinetik der GFP-Fluoreszenz der Stämme über einen Zeitraum von 8 Stunden bestimmt. Dazu werden die Stämme in einer OD₆₀₀ von 1,5 eingesetzt (Fluoreszenzmessung erfolgt wie oben).

### Inhibitions-Assay zur Identifizierung von Splicing inhibierenden Substanzen

Die Testsubstanzen werden in DMSO gelöst und in Wasser verdünnt (Endkonzentration 100 µM). Je 5 µl dieser Lösung werden in einer 384 well MTP vorgelegt. Anschließend werden 45 µl *U. maydis*-Zellen einer OD₆₀₀ von 1,25 in Minimalmedium mit 0,1% Kelzan (Fa. Monsanto) zugegeben. Danach wird die Fluoreszenz im Fluorimeter bestimmt. Nach 3 h und 6 h erfolgen weitere Messungen. Die Schwelle für eine GFP-Induktion wird beim 1,5fachen des Mittelwerts der Hintergrundfluoreszenz angesetzt.

### Erläuterungen zum Sequenzprotokoll

- **SEQ ID NO.1:**: DNA-Sequenz des Primers lga 25'
- **SEQ ID NO. 2:**: DNA-Sequenz des Primers CA52
- **SEQ ID NO. 3:**: DNA-Sequenz des Primers CA53
- **SEQ ID NO. 4:**: DNA-Sequenz des Primers 3'-GFP-Not
- **SEQ ID NO. 5:**: DNA-Sequenz des Primers lga 25'mut
- **SEQ ID NO. 6:**: DNA-Sequenz des Primers GFP5'
- **SEO ID NO. 7:**: DNA-Sequenz des Primers GFP3'
- **SEQ ID NO. 8:**: DNA-Sequenz des Primers 5'UTR
- **SEO ID NO. 9:**: DNA-Sequenz des Primers Intron
- **SEQ ID NO. 10:**: DNA-Sequenz des modifizierten Introns Nr. 1 aus dem *lga2*-Gens von *Ustilago maydis* (funktionell)
- **SEQ ID NO. 11:**: DNA-Sequenz des mutierten Introns Nr. 1 aus dem *lga2*-Gens von *Ustilago maydis* (funktionslos)
- **SEQ ID NO. 12:**: DNA-Sequenz des Primers CBX-S3
- **SEQ ID NO. 13:**: DNA-Sequenz des Primers CBX-A4
- **SEO ID NO. 14:**: DNA-Sequenz des *oma*-Promotors

### Erläuterungen zu den Abbildungen

### Abbildung 1: Schematische Darstellung der beiden Transesterifikationsschritte des Splicing-Vorgangs

A: 1. Transesterifikationsschritt:
   Innerhalb der prä-mRNA erfolgt ein nucleophiler Angriff der 2'-OH-Gruppe des invarianten Adenosin-Bausteins der Lariat-Bindungsstelle auf die 5'-Phosphatgruppe des Guanosin-Bausteins des Introns, wodurch die so genannte Lariat-Struktur entsteht.
B: 2. Transesterifikationsschritt:
   Im zweiten Schritt greift die freie 3'-OH-Gruppe des bei der ersten Reaktion abgespaltenen 5'-Exons die Phosphodiesterbindung an der 3'-Splicing-Stelle an.
C: Produkte:
   Als Ergebnis der beiden Umesterungen aus den Schritten A und B erhält man die mRNA und das Lariat-Intron.

In den Abbildungen steht A für Adenosin, G für Guanosin, C für Cytosin, U für Uracil. Y steht für T oder C. Die an der ersten Umesterung beteiligte Phosphatgruppe ist mit einem weiß hinterlegten "P" im Kreis dargestellt, die an der zweiten Umesterung beteiligte Phosphatgruppe mit einem grau hinterlegten "P" im Kreis. Angegeben sind die 5'- und 3'-Splicing-Konsensussequenzen höherer Eukaryonten.

### Abbildung 2: Schematische Darstellung der DNA-Konstrukte

A: Beispielhafter allgemeiner Aufbau eines DNA-Konstruktes, bei dessen Anwesenheit in einer eukaryontischen Zelle die Aktivität des Splicing untersucht werden kann. Ein erfindungsgemäßes DNA-Konstrukt besteht aus einem Promotor (P), einer Intron-Sequenz [(I) einschließlich der flankierenden 5'-Splicing-Stelle (5'-S) und der 3'-Splicing-Stelle (3'-S)] und dem Reporter-Gen (R).
B: Bevorzugter Aufbau eines erfindungsgemäßen DNA-Konstruktes bestehend aus dem Promotor Potef und dem modifizierten Intron Nr. 1 au dem *lga2*-Gen von *U. maydis* flankiert von der 5'-Splicing-Stelle mit der Sequenz GTAAGT und der 3'-Splicing-Stelle mit der Sequenz CAG (codierend für die Aminosäure Glutamin (Q)). Vor der 3'-Splicing-Stelle befindet sich das Startcodon AUG (codierend für die Aminosäure Methionin (M)). Als Reporter-Gen wird das eGFP-Gen verwendet.

In dieser Abbildung sind die Promotoren jeweils durch einen gepunkteten Pfeil dargestellt. Die Intron-Sequenz wird durch einen weißen Balken wiedergegeben, wobei die 5'-Splicing-Stelle durch einen waagerecht schraffierter Balken und die 3'-Splicing-Stelle durch einen senkrecht schraffierter Balken markiert sind. Das Reporter-Gen wird durch einen schwarz-weiß karierten Balken abgebildet.

### Abbildung 3: Darstellung der mRNAs in den einzelnen Splicing-Reporterstämmen

A: Durch Splicing ist das Intron in der reifen mRNA nicht mehr vorhanden. Dadurch wurde ebenso das Startcodon für die Translation von GFP entfernt. Fluoreszenz wird daher nicht beobachtet.
B: Splicing wird durch einen Inhibitor verhindert. Die mRNA enthält das Startcodon für die Translation des GFP, wodurch Fluoreszenz beobachtet wird.
C: Die Intron-Sequenz wurde in der 5'-Splicing-Stelle mutiert, wodurch auch bei Abwesenheit eines Inhibitors kein Splicing stattfindet. GFP wird immer exprimiert und die maximale Fluoreszenz kann beobachtet werden. Dieses Konstrukt dient als Positivkontrolle.

GFP wird in dieser Abbildung durch einen schwarz-weiß karierten Balken abgebildet. Die Intron-Sequenzen werden durch einen weißen Balken wiedergegeben, wobei die 5'-Splicing-Stelle durch einen waagerecht schraffierter Balken und die 3'-Splicing-Stelle durch einen senkrecht schraffierter Balken markiert sind. Die mutierte 5'-Splicing-Stelle in Abbildung C wird durch einen grauen Balken dargestellt.

### Abbildung 4: Southern-Analyse der Splicingstämme, Intergration der Konstrukte im cbx-Locus von U. maydis.

Je 2,5 µg der genomischen DNA bei Transformanden und 3 ng bei Plasmiden wurden mit *Bam*HI geschnitten, der Größe nach aufgetrennt, geblottet und mit einer für das cbx-Gen spezifischen DNA-Sonde hybridisiert.
- Spur M:: 1kb+ Größenstandard
- Spur 1:: Um518
- Spur 2:: UMA3
- Spur 3-7:: 518lga2eGFP Kandidaten
- Spur 8-11:: 518lga2muteGFP Kandidaten
- Spur 12:: plga2eGFP
- Spur 13:: plga2eGFP#2
- Spur 14:: plga2eGFP#7

### Abbildung 5: Nachweis der verschiedenen GFP-mRNA-Spezies in den Splicing-Teststämmen durch RT-PCR

A: RT-PCR Nachweis der GFP-mRNA-Spezies in den unterschiedlichen Splicing Zuständen. Die mRNA wurde aus *U. maydis* Flüssigkulturen isoliert, die 12 h in PD-Medium gewachsen waren. Für die RT-PCR-Reaktion wurden drei Primerkombinationen verwendet (5'GFP/3'GFP, oberes Drittel der Abbildung; Intron/3'GFP, mittleres Drittel der Abbildung; 5'UTR/3'GFP, unteres Drittel der Abbildung).
   Spur 1: Teststamm GFP mit Wildtyp-Intron (Stamm DS#873)
   Spur 2: Teststamm GFP mit 5'mut-Intron (Stamm DS#877)
   Spur 3: Teststamm GFP-Kontrollstamm (Stamm UMA3)
   Spur 4: 1kb+-Größenmarker
B: Schematische Darstellung der mRNA und der PCR Produkte bei aktivem Splicing (nicht maßstabsgetreu)
C: Schematische Darstellung der mRNA und der PCR Produkte bei inaktivem Splicing (nicht maßstabsgetreu)

Entsprechend Abb. 2 und Abb. 3 ist die mRNA in Abb. 5B und Abb. 5C als Balkendiagramm dargestellt. Farben und Muster entsprechen denen in Abb. 3.

Jeweils unter dem Balkendiagramm der mRNA ist durch einen Pfeil angegeben, welcher Primer an welcher Stelle ansetzt. Die verwendeten Primer sind 5'UTR, 5'GFP, 3'GFP und Intron. Die Längen der zu erwartenden RT-PCR-Produkte sind als schwarzer Balken mit Angabe der Länge in bp (base pairs) angegeben.

### Abbildung 6: Zeitabhängige Zunahme der GFP-Fluoreszenz in den Splicing-Teststämmen.

Die relative Fluoreszenz der Teststämme ist gegen die Zeit aufgetragen.

### Literatur

Berget SM, Moore C, Sharp PA (1977): Spliced segments at the 5' terminus of adenovirus 2 late mRNA. *Proc. Natl. Acad. Sci. USA* **74,** 3171-3175.

Bölker M, Urban M, Kahmann R (1992): Tha *a* mating type locus of *U. maydis* specifies cell signaling components. *Cell* **68,** 441-450.

Bottin A, Kämper J, Kahmann R (1995): Isolation of a carbon source regulated gene from *Ustilago maydis. Mol. Gen. Genet.* **253,** 342-452.

Fischer U, Liu Q, Dreyfuss G (1997): The SMN-SIP1 complex has an essential role in spliceosomal snRNP biogenesis. *Cell* **90**, 1023-1029.

Grabowski PJ, Seiler SR, Sharp PA (1985): A Multicomponent Complex Is Involved in the Splicing of Messenger RNA Precursors. *Cell* **42,** 345-353.

Holden DW, Kronstad JW, Leong SA (1989): Mutation in a heat-regulated *hsp70* gene of *Ustilago maydis. EMBO J*. **8,** 1927-1934.

Holliday R (1974): *Ustilago maydis*. In King RC (ed), Handbook of Genetics. Vol. 1, Plenum, New York, pp.575-595.

Hoffmann CS, Winston F (1987): A ten-minute DNA preparation from yeast efficiently releases autonomous plasmids for transformation in E. coli. Gene. *Gene* 267-272.

Keon JPR, White GA, Hargraves JA (1991): Isolation, charakterization and sequence of a gene conferring resistance to the systemic fungizide carboxin from the maize smut pathogene *Ustilago maydis. Curr. Genet.* **19,** 475-481.

Sambrook J (1977): Adenovirus amazes at Cold Spring Harbor. *Nature* **268,** 101-104.

Sambrook J, Fritsch EF, Maniatis T (1989): Molecular cloning: A laboratory manual. Cold Spring Harbor Laboratory Press, Cold Spring Harbor, New York.

Schmitt BE, Brown TA, Trumpower BL (1990): A rapid and simple method for preparation of RNA from *Saccharomyces cerevisiae. Nucleic Acids Res*. **18,** 3091-3092.

Schulz B, Banuett F, Dahl M, Schlesinger R, Schäfer W, Martin T, Herskowitz I, Kahmann R (1990): The *b* Alleles of *Ustilago maydis*, Whose Combinations Program Pathogenic Development, Code for Polypeptides Containing a Homeodomain-related Motif. *Cell* **60,** 295-306.

Sherman L, Sleeman J, Dall P, Hekele A, Moll J, Ponta H, Herrlich P (1996): The CD44 proteins in embryonic development and in cancer. *Curr. Top. Microbiol. Immunol.* **213,** 249-269.

Spellig T, Bottin A, Kahmann R (1996): Green fluorescent protein (GFP) as a new vital marker in the phytopathogenic fungus *Ustilago maydis. Mol. Gen. Genet.* **252,** 503-509.

Urban M, Kahmann R, Bölker M (1996): The biallelic *a* mating type locus of *Ustilago maydis*: remnants of an additional pheoromone gene indicate evolution from a multiallelic ancestor. *Mol. Gen. Genet.* **250,** 414-420.

Zanelli E, Henry M, Charvet B, Malthiery Y (1990): Evidence for an alternate splicing in the thyroperoxidase messenger from patients with Graves' disease. *Biochem. Biophys. Res. Commun.* **170,** 735-741.

## Patentansprüche

1. DNA-Konstrukt, umfassend
a) einen in Eukaryonten aktiven Promotor,
b) eine DNA-Sequenz, die alle Elemente eines funktionellen Introns besitzt, und
c) ein Reporter-Gen,
wobei alle drei Elemente in funktioneller Weise miteinander verknüpft sind.

2. DNA-Konstrukte gemäß Anspruch 1, **dadurch gekennzeichnet, dass** das Reporter-Gen in der Weise mit dem Intron verknüpft ist, dass die Entstehung des Reporter-Gen-Produktes bei korrektem Splicing gewährleistet ist.

3. DNA-Konstrukte gemäß Anspruch 1, **dadurch gekennzeichnet, dass** das Reporter-Gen in der Weise mit dem Intron verknüpft ist, dass die Entstehung des Reporter-Gen-Produktes bei gestörtem bzw. ganz unterdrücktem Splicing gewährleistet ist.

4. DNA-Konstrukt gemäß einem der Ansprüche 1 bis 3, **dadurch gekennzeichnet, dass** der Promotor aus *Ustilago maydis* stammt.

5. DNA-Konstrukt gemäß Anspruch 4, **dadurch gekennzeichnet, dass** der Promotor aus einem der folgenden Promotoren ausgewählt ist:
a) (regulierbarer) *crg1*-Promotor,
b) (konstitutiver) *hsp70*-Promotor,
c) synthetischer *otef*-Promotor,
d) synthetischer *oma*-Promotor mit der Sequenz gemäß SEQ ID NO. 14.

6. DNA-Konstrukte gemäß einem der Ansprüche 1 bis 5, **dadurch gekennzeichnet, dass** die Intron-Sequenz vom Spliceosom als Intron erkannt und bei intaktem Splicing aus der DNA herausgeschnitten wird.

7. DNA-Konstrukte gemäß Anspruch 6, **dadurch gekennzeichnet, dass** die 5'-Splicing-Stelle der Intron-Sequenz die Nucleotide 5'-GU-3' umfasst.

8. DNA-Konstrukte gemäß einem der Ansprüche 6 oder 7, **dadurch gekennzeichnet, dass** die 5'-Splicing-Stelle die Nucleotide 5'-GUAAGU-3' umfasst.

9. DNA-Konstrukte gemäß Anspruch 6, **dadurch gekennzeichnet, dass** die 3'-Splicing-Stelle der Intron-Sequenz die Nucleotide 5'-AG-3' umfasst.

10. DNA-Konstrukte gemäß einem der Ansprüche 6 oder 9, **dadurch gekennzeichnet, dass** die 3'-Splicing-Stelle die Nucleotide 5'-YAG-3' umfasst, wobei Y für eine Pyrimidinbase steht.

11. DNA-Konstrukte gemäß einem der Ansprüche 6 bis 10, **dadurch gekennzeichnet, dass** die Intron-Sequenz keine Start- und/oder Stoppcodons enthält.

12. DNA-Konstrukt gemäß einem der Ansprüche 6 bis 11, **dadurch gekennzeichnet, dass** die Intron-Sequenz ausgewählt ist aus einem der vier Introns des *lga2*-Gens und einem der drei Introns aus dem *pra1*-Gen aus *Ustilago maydis*.

13. DNA-Konstrukt gemäß Anspruch 12, **dadurch gekennzeichnet, dass** das Intron der Sequenz gemäß SEQ ID NO. 10 entspricht.

14. DNA-Konstrukt gemäß einem der Ansprüche 1 bis 13, **dadurch gekennzeichnet, dass** es ein Reporter-Gen aus der folgenden Gruppe von Reporter-Genen umfasst: GFP und dessen Varianten und dessen Derivate (z.B. eGFP, yGFP, cGFP), lacZ, LUX, GUS, CAT, Orotidin 5'-Monophosphatdecarboxylase, Nitrat Reduktase.

15. DNA-Konstrukt gemäß Anspruch 14, **dadurch gekennzeichnet, dass** das Reporter-Gen eGFP ist.

16. DNA-Konstrukt gemäß einem der Ansprüche 1 bis 15, bestehend aus dem *otef*-Promotor, einem Intron mit einer Sequenz gemäß SEQ ID NO. 10 und dem eGFP-Gen.

17. DNA-Konstrukt gemäß einem der Ansprüche 1 bis 15 bestehend aus dem *oma*-Promotor mit einer Sequenz gemäß SEQ ID NO. 14, dem Intron mit einer Sequenz gemäß SEQ ID NO. 10 und dem eGFP-Gen.

18. Verfahren zum Herstellen eines DNA-Konstruktes gemäß Anspruch 1, **dadurch gekennzeichnet ist, dass** man
a) in einem ersten Schritt
i) ein geeignetes Intron amplifiziert, wobei die Sequenz des Introns gegebenenfalls durch die Wahl eines geeigneten Primers gezielt modifiziert wird,
ii) ein geeignetes Reporter-Gen amplifiziert,
b) in einem zweiten Schritt die beiden Amplifikate aus Schritt (a) unabhängig voneinander in ein geeignetes Plasmid I kloniert, wodurch man die beiden Plasmide II (Intron) und III (Reporter-Gen) erhält,
c) in einem dritten Schritt
i) aus dem Plasmid II (Intron) das Intronfragment mit geeigneten Restriktionsenzymen herausschneidet,
ii) aus dem Plasmid III (Reporter-Gen) das Reporter-Gen-Fragment mit geeigneten Restriktionsenzymen herausschneidet,
iii) einen geeigneten Vektor restringiert und
iv) die drei erhaltenen Fragmente so ligiert, dass man ein Plasmid V erhält, in dem die Intron-Sequenz und das Reporter-Gen funktionell verknüpft sind.

19. Wirtszellen und Wirtsorganismen, **dadurch gekennzeichnet, dass** sie DNA-Konstrukte gemäß einem der Ansprüche 1 bis 17 enthalten.

20. Wirtszellen und Wirtsorganismen gemäß Anspruch 19, **dadurch gekennzeichnet, dass** es sich um eukaryontischen Zellen, Zelllinien von Säugern oder Pilzen handelt.

21. Wirtszellen und Wirtsorganismen gemäß einem der Ansprüche 19 oder 20, **dadurch gekennzeichnet, dass** es sich um Zellen von Pilzen, Insekten, Pflanzen, Froschoozyten sowie Volvox-Kugeln, Drosophila-Embryonen oder Daphnia-Larven handelt.

22. Wirtszellen und Wirtsorganismen gemäß Anspruch 21, **dadurch gekennzeichnet, dass** es sich um Zellen von Pilzen handelt.

23. Wirtszellen und Wirtsorganismen gemäß Anspruch 22, **dadurch gekennzeichnet, dass** es sich um Zellen von *Saccharomyces cerevisiae, Magnaporthe grisea, Aspergillus nidulans, Cochliobulus heterostrophus, Nectria hematococca, Botrytis cinerea, Gaeumannomyces sp., Pichia pastoris* und *Ustilago maydis* handelt.

24. Wirtszellen und Wirtsorganismen gemäß Anspruch 23, **dadurch gekennzeichnet, dass** es sich um *Ustilago maydis* handelt.

25. Verfahren zum Nachweis der Funktionsfähigkeit des Splicing *in vivo.*

26. Verfahren gemäß Anspruch 25, **dadurch gekennzeichnet, dass** man
(A) ein DNA-Konstrukt gemäß einem der Ansprüche 1 bis 17 herstellt,
(B) dieses DNA-Konstrukt in eine Wirtszelle oder einen Wirtsorganismus einbringt, und
(C) die Anwesenheit oder Abwesenheit des Reporter-Gen-Produktes kontrolliert

27. Verfahren gemäß Anspruch 26, **dadurch gekennzeichnet, dass** man
(A) ein DNA-Konstrukt gemäß Anspruch 3 herstellt,
(B) dieses DNA-Konstrukt in eine Wirtszelle oder einen Wirtsorganismus einbringt, und
(C) die Anwesenheit des Reporter-Gen-Produktes kontrolliert.

28. Verfahren zum Identifizieren von Inhibitoren des Splicing, **dadurch gekennzeichnet, dass** man
(a) ein DNA-Konstrukt gemäß einem der Ansprüche 1 bis 17 herstellt;
(b) das DNA-Konstrukt aus Schritt (a) in eine Wirtszelle oder einen Wirtsorganismus einbringt;
(c) die Wirtszelle oder den Wirtsorganismus aus Schritt (b) mit einer Einzelsubstanz oder einer Mischung von mehreren chemischen Verbindungen in Kontakt bringt,
(d) die Anwesenheit oder Abwesenheit des Reporter-Gen-Produktes in Gegenwart der Einzelsubstanz oder einer Mischung von mehreren chemischen Verbindungen vergleicht mit der Anwesenheit oder Abwesenheit des Reporter-Gen-Produktes bei deren Abwesenheit, und
(e) gegebenenfalls die Verbindung oder Verbindungen identifiziert, wodurch die Funktionalität des Splicing beeinflusst wird.

29. Verfahren gemäß Anspruch 28, **dadurch gekennzeichnet, dass** man
(a) ein DNA-Konstrukt gemäß Anspruch 3 herstellt;
(b) das DNA-Konstrukt aus Schritt (a) in eine Wirtszelle oder einen Wirtsorganismus einbringt;
(c) die Wirtszelle oder den Wirtsorganismus aus Schritt (b) mit einer Einzelsubstanz oder einer Mischung von mehreren chemischen Verbindungen in Kontakt bringt,
(d) die Anwesenheit des Reporter-Gen-Produktes in Gegenwart der Einzelsubstanz oder einer Mischung von mehreren chemischen Verbindungen vergleicht mit der Anwesenheit des Reporter-Gen-Produktes bei deren Abwesenheit, und
(e) gegebenenfalls die Verbindung oder Verbindungen identifiziert, wodurch die Funktionalität des Splicing beeinflusst wird.

30. Verfahren zum Identifizieren von Verbindungen, welche die Expression von Komponenten des Splicing-Apparates beeinflussen, **dadurch gekennzeichnet, dass** man
(a) ein DNA-Konstrukt gemäß einem der Ansprüche 1 bis 17 herstellt;
(b) das DNA-Konstrukt aus Schritt (a) in eine Wirtszelle oder einen Wirtsorganismus einbringt;
(c) die Wirtszelle oder den Wirtsorganismus aus Schritt (b) mit einer Einzelsubstanz oder einer Mischung von mehreren chemischen Verbindungen in Kontakt bringt,
(d) die Anwesenheit oder Abwesenheit des Reporter-Gen-Produktes in Gegenwart der Einzelsubstanz oder einer Mischung von mehreren chemischen Verbindungen vergleicht mit der Anwesenheit oder Abwesenheit des Reporter-Gen-Produktes bei deren Abwesenheit,
(e) die Polypeptid- und/oder RNA-Zusammensetzung des Spliceosoms bestimmt, und
(f) gegebenenfalls die Verbindung oder Verbindungen identifiziert, wodurch die Funktionalität des Splicing beeinflusst wird.

31. Verfahren gemäß einem der Ansprüche 26 bis 30, **dadurch gekennzeichnet, dass** man Wirtszellen oder Wirtsorganismen gemäß einem der Ansprüche 19 bis 24 verwendet.

32. Verwendung eines DNA-Konstruktes gemäß einem der Ansprüche 1 bis 17 in Verfahren gemäß einem der Ansprüche 26 bis 31.

33. Verwendung des Splicing in Verfahren zum Identifizieren von fungiziden, insektiziden und/oder herbiziden Verbindungen.

34. Verwendung von Modulatoren des Splicing als Fungizide oder Antimycotica.

35. Verwendung von Modulatoren des Splicing als Insektizide oder Herbizide.

36. Modulatoren des Splicing, die mit Hilfe eines Verfahrens gemäß einem der Ansprüche 26 bis 31 gefunden werden.


SEQUENZPROTOKOLL

<110>  Bayer AG

<120>  Splicing als Target zum Identifizieren neuer Wirkstoffe

<130>  LeA 35262

<160>  14

<170>  PatentIn version 3.1

<210>  1
<211>  28
<212>  DNA
<213>  Primer lga25'

<400>  1
gccagatcta ggtaagttgc ttcaaatc                                        28

<210>  2
<211>  26
<212>  DNA
<213>  Primer CA52

<400>  2
ctgcatgcga aaatgaaaag tcgacg                                          26

<210>  3
<211>  28
<212>  DNA
<213>  Primer CA53

<400>  3
tagcatgcag gtgagcaagg gcgaggag                                          28

<210>  4

<211>  25

<212>  DNA

<213>  Primer 3'-GFP-Not

<400>  4
agcggccgct tacttgtaca gctcg                                             25

<210>  5

<211>  28

<212>  DNA

<213>  Primer lga 25'mut

<400>  5
gccagatcta cttaagttgc ttcaaatc                                          28

<210>  6

<211>  18

<212>  DNA

<213>  Primer GFP5'

<400>  6
gtgagcaagg gcgaggag                                                     18

<210>  7

<211>  21

<212>  DNA

<213>  Primer GFP3'

<400>  7
ctagattact tgtacagctc g                                                 21

<210>  8

<211>  20

<212>  DNA

<213>  Primer 5'UTR

<400>  8
cacagacaac atcatccacg                                                    20

<210>  9

<211>  21

<212>  DNA

<213>  Primer Intron

<400>  9
tgcttcaaat cagattacac t                                                  21

<210>  10

<211>  78

<212>  DNA

<213>  modifiziertes lga2-Intron

<400>  10
gatctaggta agttgcttca aatcagatta cactggataa gaacatatct gacgtcgact      60

tttcattttc gcatgcag                                                      78

<210>  11

<211>  78

<212>  DNA

<213>  mutiertes lga2-Intron

<400>  11
gatctactta agttgcttca aatcagatta cactggataa gaacatatct gacgtcgact      60

tttcattttc gcatgcag                                                      78

<210>  12

<211>  23

<212>  DNA

<213>  Primer CBX-S3

<400>  12
agtcgtacac ctggacctca acc                                              23

<210>  13

<211>  21

<212>  DNA

<213>  Primer CBX-A4

<400>  13
ggctcgatgg atcggtactg c                                                21

<210>  14

<211>  1338

<212>  DNA

<213>  oma-Promotor

<400>  14
tcgagtgcca cacttgtcac aatacgcagg aaccgccgtt cgcacactat acgttggtgt      60

ggtcttgcaa atatgcacac cgtccatcaa gcttatcgat accgtcgagt gccacacttg     120

tcacaatacg caggaaccgc cgttcgcaca ctatacgttg gtgtggtctt gcaaatatgc     180

acaccgtcca tcaagcttat cgataccgtc gagtgccaca cttgtcacaa tacgcaggaa     240

ccgccgttcg cacactatac gttggtgtgg tcttgcaaat atgcacaccg tccatcaagc     300

ttatcgatac cgtcgaggtc gagtgccaca cttgtcacaa tacgcaggaa ccgccgttcg     360

cacactatac gttggtgtgg tcttgcaaat atgcacaccg tccatcaagc ttatcgatac     420

cgtcgagtgc cacacttgtc acaatacgca ggaaccgccg ttcgcacact atacgttggt     480

gtggtcttgc aaatatgcac accgtccatc aagcttatcg ataccgtcga gtgccacact     540

tgtcacaata cgcaggaacc gccgttcgca cactatacgt tggtgtggtc ttgcaaatat     600

gcacaccgtc catcaagctt atcgataccg tcgaggtcga gtgccacact tgtcacaata     660

cgcaggaacc gccgttcgca cactatacgt tggtgtggtc ttgcaaatat gcacaccgtc     720

catcaagctt atcgataccg tcgagtgcca cacttgtcac aatacgcagg aaccgccgtt     780

cgcacactat acgttggtgt ggtcttgcaa atatgcacac cgtccatcaa gcttatcgat     840

accgtcgagt gccacacttg tcacaatacg caggaaccgc cgttcgcaca ctatacgttg 900

gtgtggtctt gcaaatatgc acaccgtcca tcaagcttat cgataccgtc gaggtcgacg 960

gtatcgataa gcttgatatc gaattgatcc cggtcacctt cctggatgag aagaccaact 1020

tcgattacta tgtctgcgca gggaaaggtg taactgctgg ctgctcagtg tacgattgtc 1080

gaagaagcat ctcgggatgt cagcactctt actcacctgg tgcgttgcgc tcatgagccc 1140

ttgagacaag cgaagtccat cttctgcaac gcaatgctcg acatcactga gacggtaccg 1200

tcaaggatat aagggagcaa ttggatatca atccgacagc caaacctcat ccactctcac 1260

tttcacactc taacttatac gatcacttct cgcccgttct tttgaacatc aaatcaacta 1320

ccttactcta tcaggatc 1338